# EUROPEAN PATENT APPLICATION

(11) **EP 4 600 352 A1**
(43) Date of publication of application: **13.08.2025**
(21) Application number: 23872234.2
(22) Date of filing: 25.09.2023
(51) Int. Cl.: C12N 5/0783, A61K 35/17, A61P 37/06, C12N 5/10, C12N 15/12

(54) **T CELL PRODUCTION METHOD**

(30) Priority: 26.09.2022 JP 2022152606
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); IRIGUCHI, Shoichi, Kyoto-shi, Kyoto 606-8501 (JP); SATO, Takayuki, Fujisawa-shi, Kanagawa 251-0012 (JP); KASSAI, Yoshiaki, Fujisawa-shi, Kanagawa 251-0012 (JP)
(74) Representative: Jones, Nicholas Andrew
(86) International application number: PCT/JP2023/034652
(87) International publication number: WO 2024/071010

(57) **Abstract**

Disclosed are a method for producing a cell population in which regulatory T cells have proliferated, including (1) culturing a cell population containing CD4⁺ T cells derived from pluripotent stem cells in the presence of IL-4 and a TGF-βR agonist, a cell population containing regulatory T cells obtained according to the method, and a medicine containing the cell population containing regulatory T cells.

## Description

### Technical Field

The present invention relates to a method for producing a cell population in which regulatory T cells have proliferated, a cell population containing regulatory T cells obtained according to the method, a medicine containing the cell population containing regulatory T cells, and the like.

### Background of Invention

Due to the immune response suppression function of regulatory T cells (Tregs), cell therapies using regulatory T cells (Treg therapies) have been increasingly studied and developed worldwide with the aim of treating various autoimmune diseases. Regulatory T cells show promise for use in the treatment of graft-versus-host disease (GvHD) and autoimmune diseases, or in the treatment and prevention of inflammatory diseases and allergic diseases.

Regulatory T cells are roughly classified into two types: naturally occurring regulatory T cells (natural Treg: nTreg or thymic Treg: tTreg) and inducible regulatory T cells (induced Treg: iTreg), and it is known that nTregs naturally occur in the thymus, and that iTregs differentiate from naive T cells in peripheral blood by induction by antigen stimulation and cytokines such as IL-2 and TGF-β. These regulatory T cells are further subdivided according to the type of marker to be expressed in the cells.

When regulatory T cells are depleted in vivo, various organ-specific autoimmune diseases develop spontaneously, and at that time, when regulatory T cells are transplanted, the development of autoimmune diseases is prevented. Accordingly, regulatory T cells have been considered to play an important role in maintaining peripheral immune self-tolerance. Since then, it has become clear that regulatory T cells can suppress not only autoimmunity but also most immune responses, such as inflammation due to foreign antigens, rejection due to transplantation, infectious immunity, allergies, and tumor immunity. Further, currently, the transcription factor FOXP3 has been revealed as a master regulator of the regulatory T cells.

FOXP3 is a master regulator of Tregs, and it is known that FOXP3 is constitutively expressed in Tregs, and that constitutive expression is not observed in normal T cells having no suppressive function (which may also be referred to as Tconv (conventional T cells), effector T cells, or inflammatory T cells). Therefore, studies on the maintenance of FOXP3 expression in primary Tregs and the induction of FOXP3 expression in primary Tconv or bulk CD4 single-positive (SP) T cells have been conducted, and such compounds and cytokines having the activity of maintaining and inducing FOXP3 expression, and combinations thereof have been reported. Such compounds and cytokines include IL-3 (NPL 1), TGF-β (NPL 2 and 3), and IL-4 (NPL 4 and 5).

Furthermore, such compounds, cytokines, and combinations thereof include AS2863619 as a CDK8/19 inhibitor (NPL 6), rapamycin as an mTOR inhibitor (NPL 7, NPL 8, NPL 9, and NPL 10), a TNFR2 agonist antibody (NPL 9, NPL 11, and NPL 12), cytokine TGF-β (NPL 6 and NPL 10), and the like.

While the effects of IL-3, IL-4, and TGF-β on primary Tregs have been reported as mentioned above, there are no reports indicating that the combination of IL-4 and TGF-β (and further including IL-3) is particularly preferable. Regarding IL-4, there are mixed reports on whether it is advantageous or disadvantageous for Treg differentiation and functionality. Moreover, NPL 3 reports that adding IL-4 leads to completely different effects from those of TGF-β, with IL-4 suppressing FOXP3 expression in mouse Tregs and Treg proliferation.

For pluripotent stem cell-derived regulatory T cells, compounds and cytokines maintaining FOXP3 expression, and combinations thereof; compounds and cytokines inducing FOXP3 expression from T cells not expressing FOXP3, and combinations thereof; and the like are not known. The literature noted above also does not disclose them. It is known that regulatory T cells are difficult to proliferate, especially while maintaining their suppressive function, although an industrial production method with high production efficiency (yield) is desired, and an efficient production method thereof is expected.

Regarding IPEX (immune dysregulation, polyendocrinopathy, enteropathy, X-linked) syndrome caused by a mutation in FOXP3, NPL 13 discloses that a gene was transduced into autologous hematopoietic stem cells using a lentiviral vector to restore FOXP3 expression, and that the cells were administered to a model mouse of IPEX syndrome. NPL 13 reports that as a result, hematopoietic stem cells differentiated into functional regulatory T cells and recovered from the autoimmune phenotype. Further, PTL 1 also describes a similar recombinant lentiviral vector containing a nucleotide sequence encoding a human FOXP3 protein.

PTL 2 discloses that it is important to allowing cells to express Mcl-1 together with Foxp3 for improving Treg survival. Further, PTL 3 discloses genetically engineered mammalian cells containing a transgene encoding a lineage commitment factor that promotes differentiation into CD4⁺ Tregs. PTL 4 discloses a method for preparing a composition of T cells from stem cells or progenitor cells by culturing a three-dimensional cell aggregate including a selected population of stromal cells expressing a Notch ligand and a selected population of stem cells or progenitor cells.

### Citation List

### Patent Literature

PTL 1: WO 2019/040655
PTL 2: WO 2014/180943
PTL 3: WO 2021/092581
PTL 4: WO 2017/075389

### Non-patent Literature

NPL 1: J. Immunol. (2011) 186: 2262-2272
NPL 2: J. Exp. Med. (2003) 198(12): 1875-1886
NPL 3: Immunology (2009) 128: e670-e678
NPL 4: Front. Immunol. 8: 1508. doi: 10.3389/fimmu.2017.01508
NPL 5: Immunology. 2009 Jul; 127(3): 338-344
NPL 6: Sci. Immunol. 4, eaaw2707 (2019)
NPL 7: Nat. Immunol. 20(9), 1208-1219 (2019)
NPL 8: Clin. Exp. Immunol., 197(1): 52-63. (2019)
NPL 9: Front. Immunol. 9: 573 (2018)
NPL 10: PLoS One, 11(2): e0148474. (2016)
NPL 11: PLoS One, 11(5): e0156311 (2016)
NPL 12: Sci. Signal. 13, eaba9600 (2020)
NPL 13: Cell Stem Cell 24, 309-317, February 7, 2019

### Summary of Invention

### Technical Problem

An object of the present invention is to provide a method for producing a cell population in which regulatory T cells have proliferated, capable of proliferating regulatory T cells with high efficiency while maintaining their suppressive function.

### Solution to Problem

As a result of extensive research to achieve the object, the present inventors found that culturing a cell population containing CD4⁺ T cells derived from pluripotent stem cells (in particular, iPS cells) in the presence of IL-4 and a TGF-βR agonist allows regulatory T cells to grow with high efficiency while maintaining their suppressive function.

The present invention was completed as a result of further research based on the finding and provides the following method for producing a cell population in which regulatory T cells have proliferated, cell population containing regulatory T cells, medicine, and the like.
[1] A method for producing a cell population in which regulatory T cells have proliferated, comprising:
   (1) culturing a cell population containing CD4⁺ T cells derived from pluripotent stem cells in the presence of IL-4 and a TGF-βR agonist.
[2] The method according to [1], wherein step (1) is performed in the presence of IL-4, a TGF-βR agonist, and IL-3.
[3] The method according to [1], wherein step (1) is performed in the presence of IL-4, a TGF-βR agonist, and IL-33.
[4] The method according to [1], wherein step (1) is performed in the presence of IL-4, a TGF-βR agonist, IL-3, and IL-33.
[5] The method according to any one of [1] to [4], wherein step (1) is further performed in the presence of at least one selected from the group consisting of IL-2, a TNFR2 agonist, and an mTOR inhibitor.
[6] The method according to [5], wherein step (1) is further performed in the presence of IL-2, a TNFR2 agonist, and an mTOR inhibitor.
[7] The method according to any one of [1] to [6], wherein step (1) is performed in the presence of IL-4, a TGF-βR agonist, IL-3, IL-33, IL-2, a TNFR2 agonist, and an mTOR inhibitor.
[8] The method according to any one of [1] to [7], wherein step (1) is further performed in the presence of a CDK8 and/or CDK19 inhibitor.
[9] The method according to any one of [1] to [8], wherein the TGF-βR agonist is TGF-β.
[10] The method according to any one of [5] to [9], wherein the mTOR inhibitor is rapamycin.
[11] The method according to any one of [5] to [10], wherein the TNFR2 agonist is a TNFR2 agonist antibody.
[12] The method according to any one of [8] to [11], wherein the CDK8 and/or CDK19 inhibitor is at least one selected from the group consisting of 4-[1-(2-methyl-1H-benzimidazol-5-yl)-1H-imidazo[4,5-c]pyridin-2-yl]-1,2,5-oxadiazole-3-amine, 3-{1-[1-(4-methoxyphenyl)piperidin-4-yl]-4-methyl-1H-imidazo[4,5-c]pyridin-2-yl}pyrazine-2-amine, and siRNA of CDK8 and/or CDK19.
[13] The method according to any one of [1] to [12], wherein the regulatory T cells are CD25⁺/FOXP3⁺ cells.
[13a] The method according to any one of [1] to [12], wherein the regulatory T cells are CD25⁺/CD127⁻ cells.
[13b] The method according to any one of [1] to [13a], wherein the regulatory T cells are Helios⁺ cells.
[13c] The method according to any one of [1] to [13b], wherein the regulatory T cells are CTLA4⁺ cells.
[13d] The method according to any one of [1] to [13c], wherein the regulatory T cells are CD39⁺ cells.
[13e] The method according to any one of [1] to [13d], wherein the regulatory T cells are CD73⁺ cells.
[14] The method according to any one of [1] to [13e], wherein the CD4⁺ T cells are at least one type of cells selected from the group consisting of CD25⁺/FOXP3⁺ cells and CD25⁻/FOXP3⁻cells.
[15] The method according to any one of [1] to [14], wherein the CD4⁺ T cells have an expression construct introduced, the expression construct comprising
   (a) conserved non-coding sequence (CNS) 1, CNS2, and CNS3 of FOXP3 gene,
   (b) a promoter, and
   (c) a nucleic acid encoding FOXP3.
[16] The method according to any one of [1] to [15], further comprising before step (1)
   (2) inducing differentiation of pluripotent stem cells into a cell population containing CD4⁺ T cells.
[17] The method according to [16], further comprising before step (2)
   (3) introducing an expression construct into pluripotent stem cells,
   the expression construct comprising
   (a) CNS1, CNS2, and CNS3 of FOXP3 gene,
   (b) a promoter, and
   (c) a nucleic acid encoding FOXP3.
[17a] The method according to any one of [1] to [17], comprising (4) introducing a foreign gene (in particular, a gene for expressing a chimeric antigen receptor) into pluripotent stem cells (in particular, iPS cells), a cell population containing CD4⁺ T cells, or a cell population containing regulatory T cells.
[18] A method for proliferating regulatory T cells, comprising (1A) culturing a cell population containing regulatory T cells derived from pluripotent stem cells in the presence of IL-4 and a TGF-βR agonist.
[18a] The method according to [18], wherein step (1) is performed in the presence of IL-4, a TGF-βR agonist, and IL-3.
[18b] The method according to [18], wherein step (1) is performed in the presence of IL-4, a TGF-βR agonist, and IL-33.
[18c] The method according to [18], wherein step (1) is performed in the presence of IL-4, a TGF-βR agonist, IL-3, and IL-33.
[18d] The method according to any one of [18] to [18c], wherein step (1) is further performed in the presence of at least one selected from the group consisting of IL-2, a TNFR2 agonist, and an mTOR inhibitor.
[18e] The method according to [18d], wherein step (1) is further performed in the presence of IL-2, a TNFR2 agonist, and an mTOR inhibitor.
[18f] The method according to any one of [18] to [18e], wherein step (1) is performed in the presence of IL-4, a TGF-βR agonist, IL-3, IL-33, IL-2, a TNFR2 agonist, and an mTOR inhibitor.
[18g] The method according to any one of [18] to [18f], wherein step (1) is further performed in the presence of a CDK8 and/or CDK19 inhibitor.
[18h] The method according to any one of [18] to [18g], wherein the TGF-βR agonist is TGF-β.
[18i] The method according to any one of [18d] to [18h], wherein the mTOR inhibitor is rapamycin.
[18j] The method according to any one of [18d] to [18i], wherein the TNFR2 agonist is a TNFR2 agonist antibody.
[18k] The method according to any one of [18g] to [18j], wherein the CDK8 and/or CDK19 inhibitor is at least one selected from the group consisting of 4-[1-(2-methyl-1H-**benzoimidazol-5-yl)-1H-imidazo[4,5-c]pyridin-2-yl]-1,2,5-**oxadiazole-3-amine, 3-{1-[1-(4-methoxyphenyl)piperidin-4-yl]-4-methyl-1H-imidazo[4,5-c]pyridin-2-yl}pyrazine-2-amine, and siRNA of CDK8 and/or CDK19.
[18l] The method according to any one of [18] to [18k], wherein the regulatory T cells are CD25⁺/FOXP3⁺ cells.
[18m] The method according to any one of [18] to [18l], wherein the regulatory T cells are CD25⁺/CD127⁻ cells.
[18n] The method according to any one of [18] to [18m], wherein the regulatory T cells are Helios⁺ cells.
[18o] The method according to any one of [18] to [18n], wherein the regulatory T cells are CTLA4⁺ cells.
[18p] The method according to any one of [18] to [18o], wherein the regulatory T cells are CD39⁺ cells.
[18q] The method according to any one of [18] to [18p], wherein the regulatory T cells are CD73⁺ cells.
[18r] The method according to any one of [18] to [18q], wherein the regulatory T cells derived from pluripotent stem cells are cells having an expression construct introduced, the expression construct containing
   (a) conserved non-coding sequence (CNS) 1, CNS2, and CNS3 of FOXP3 gene,
   (b) a promoter, and
   (c) a nucleic acid encoding FOXP3.
[18s] The method according to any one of [18] to [18r], further comprising before step (1)
   (2) inducing differentiation of pluripotent stem cells into a cell population containing regulatory T cells.
[18t] The method according to [18s], further comprising before step (2)
   (3) introducing an expression construct into pluripotent stem cells,
   the expression construct comprising
   (a) CNS1, CNS2, and CNS3 of FOXP3 gene,
   (b) a promoter, and
   (c) a nucleic acid encoding FOXP3.
[18u] The method according to any one of [18] to [18t], comprising (4) introducing a foreign gene (in particular, a gene for expressing a chimeric antigen receptor) into pluripotent stem cells (in particular, iPS cells) or a cell population containing regulatory T cells.
[19] A method for producing a cell population containing regulatory T cells, comprising (1B) culturing a cell population containing CD4⁺/CD25⁻/FOXP3⁻ T cells derived from pluripotent stem cells in the presence of IL-4 and a TGF-βR agonist.
[19a] The method according to [19], wherein step (1) is performed in the presence of IL-4, a TGF-βR agonist, and IL-3.
[19b] The method according to [19], wherein step (1) is performed in the presence of IL-4, a TGF-βR agonist, and IL-33.
[19c] The method according to [19], wherein step (1) is performed in the presence of IL-4, a TGF-βR agonist, IL-3, and IL-33.
[19d] The method according to any one of [19] to [19c], wherein step (1) is further performed in the presence of at least one selected from the group consisting of IL-2, a TNFR2 agonist, and an mTOR inhibitor.
[19e] The method according to [19d], wherein step (1) is further performed in the presence of IL-2, a TNFR2 agonist, and an mTOR inhibitor.
[19f] The method according to any one of [19] to [19e], wherein step (1) is performed in the presence of IL-4, a TGF-βR agonist, IL-3, IL-33, IL-2, a TNFR2 agonist, and an mTOR inhibitor.
[19g] The method according to any one of [19] to [19f], wherein step (1) is further performed in the presence of a CDK8 and/or CDK19 inhibitor.
[19h] The method according to any one of [19] to [19g], wherein the TGF-βR agonist is TGF-β.
[19i] The method according to any one of [19d] to [19h], wherein the mTOR inhibitor is rapamycin.
[19j] The method according to any one of [19d] to [19i], wherein the TNFR2 agonist is a TNFR2 agonist antibody.
[19k] The method according to any one of [19g] to [19j], wherein the CDK8 and/or CDK19 inhibitor is at least one selected from the group consisting of 4-[1-(2-methyl-1H-benzoimidazol-5-yl)-1H-imidazo[4,5-c]pyridin-2-yl]-1,2,5-oxadiazole-3-amine, 3-{1-[1-(4-methoxyphenyl)piperidin-4-yl]-4-methyl-1H-imidazo[4,5-c]pyridin-2-yl}pyrazine-2-amine, and siRNA of CDK8 and/or CDK19.
[19l] The method according to any one of [19] to [19k], wherein the regulatory T cells are CD25⁺/FOXP3⁺ cells.
[19m] The method according to any one of [19] to [19k], wherein the regulatory T cells are CD25⁺/CD127⁻ cells.
[19n] The method according to any one of [19] to [19m], wherein the regulatory T cells are Helios⁺ cells.
[19o] The method according to any one of [19] to [19n], wherein the regulatory T cells are CTLA4⁺ cells.
[19p] The method according to any one of [19] to [19o], wherein the regulatory T cells are CD39⁺ cells.
[19q] The method according to any one of [19] to [19p], wherein the regulatory T cells are CD73⁺ cells.
[19r] The method according to any one of [19] to [19q], further comprising before step (1)
   (2) inducing differentiation of pluripotent stem cells into a cell population containing CD4⁺/CD25⁻/FOXP3⁻ T cells.
[19s] The method according to any one of [19] to [19r], comprising (4) introducing a foreign gene (in particular, a gene for expressing a chimeric antigen receptor) into pluripotent stem cells (in particular, iPS cells), a cell population containing CD4⁺/CD25⁻/FOXP3⁻ T cells, or a cell population containing regulatory T cells.
[20] The method according to any one of [1] to [19s], wherein the pluripotent stem cells are iPS cells.
[21] A cell population containing regulatory T cells, obtained according to the method of any one of [1] to [20].
[22] A medicine comprising the cell population containing regulatory T cells of [21].
[23] The medicine according to [22], for use in prevention and/or treatment of an abnormally enhanced immune response.
[24] A method for preventing and/or treating an abnormally enhanced immune response, comprising administering the cell population containing regulatory T cells of [21] to a subject in need thereof.
[25] The cell population containing regulatory T cells according to [21], for use in prevention and/or treatment of an abnormally enhanced immune response.
[26] Use of the cell population containing regulatory T cells of [21] in the production of a medicine for use in prevention and/or treatment of an abnormally enhanced immune response.

### Advantageous Effects of Invention

The present invention enables regulatory T cells to proliferate with high efficiency while maintaining their suppressive function.

### Brief Description of Drawings

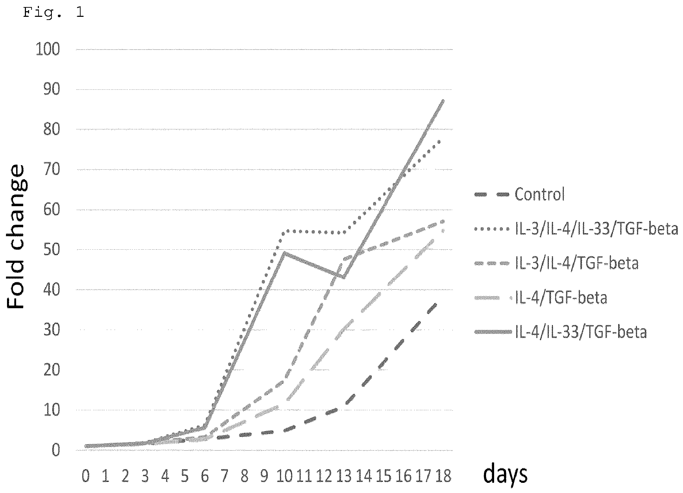

Fig. 1 shows changes in cell count over time when cells that have undergone differentiation induction from iPS cells into Tregs were expanded by culture in a medium containing IL-4 and TGF-β1.

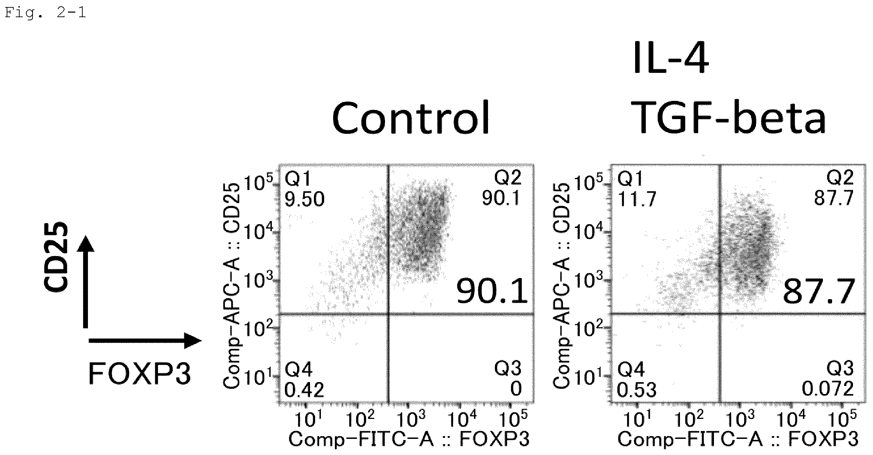

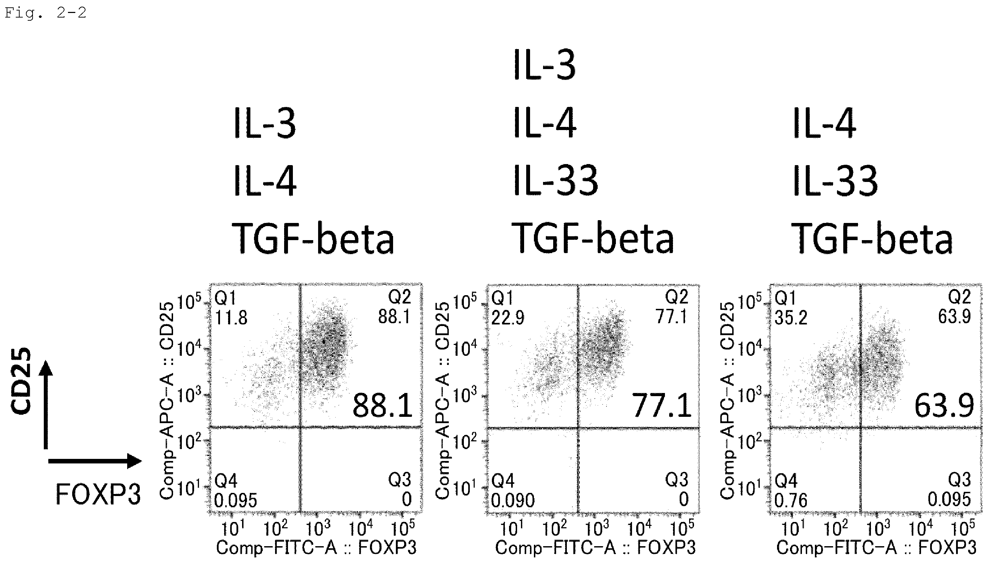

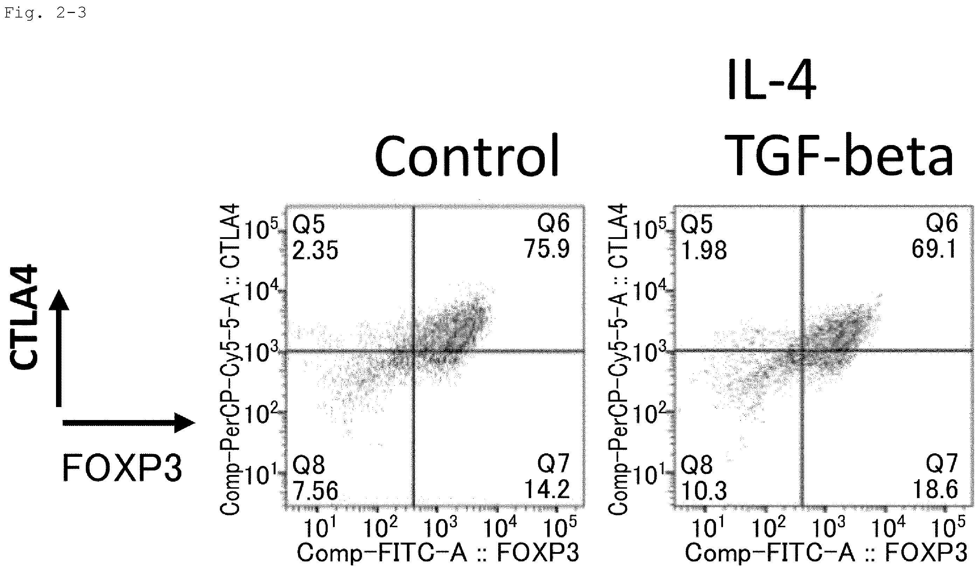

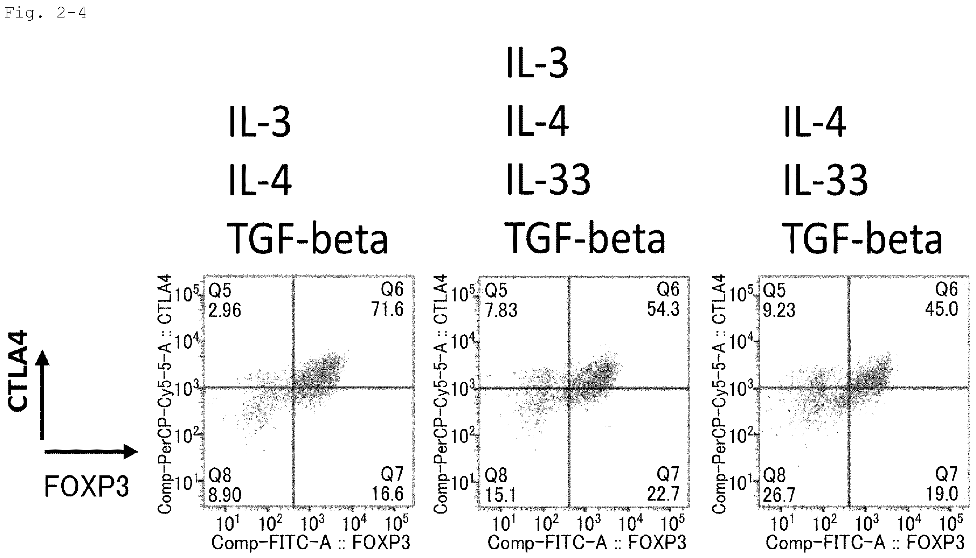

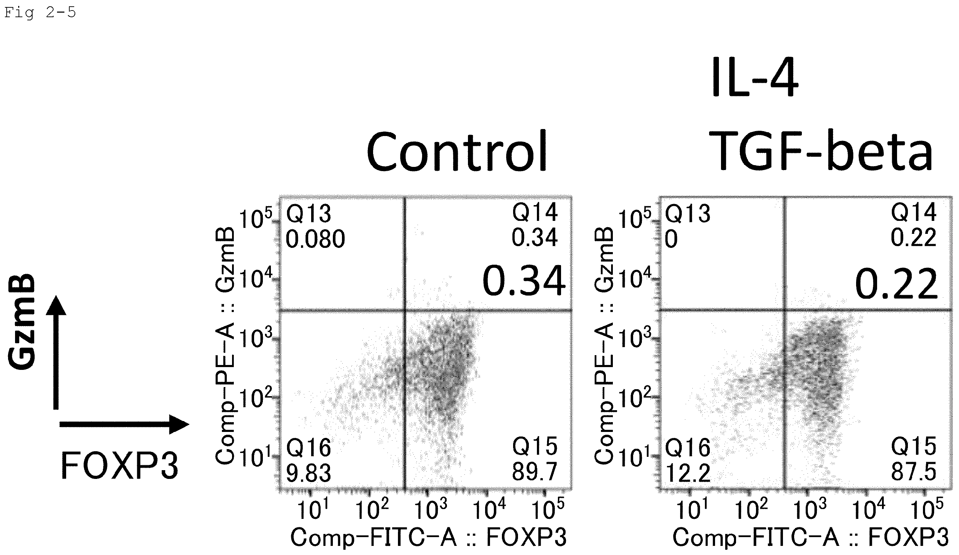

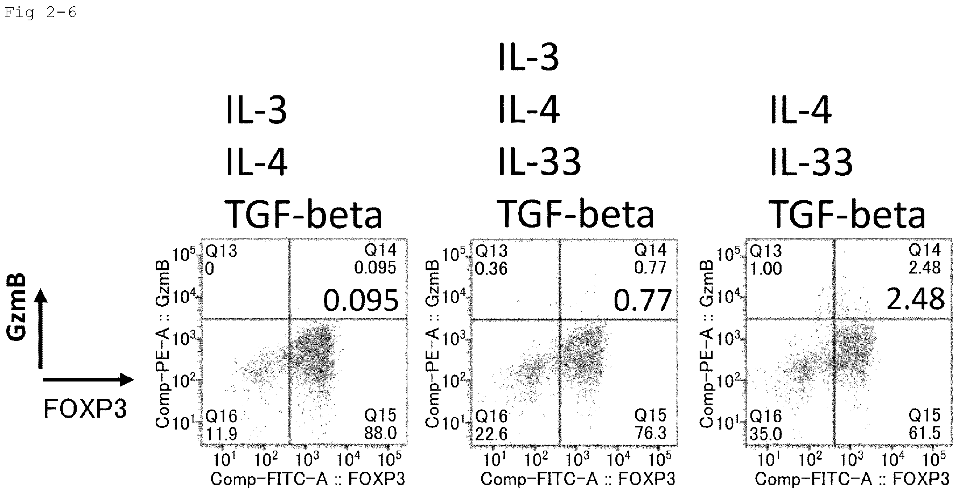

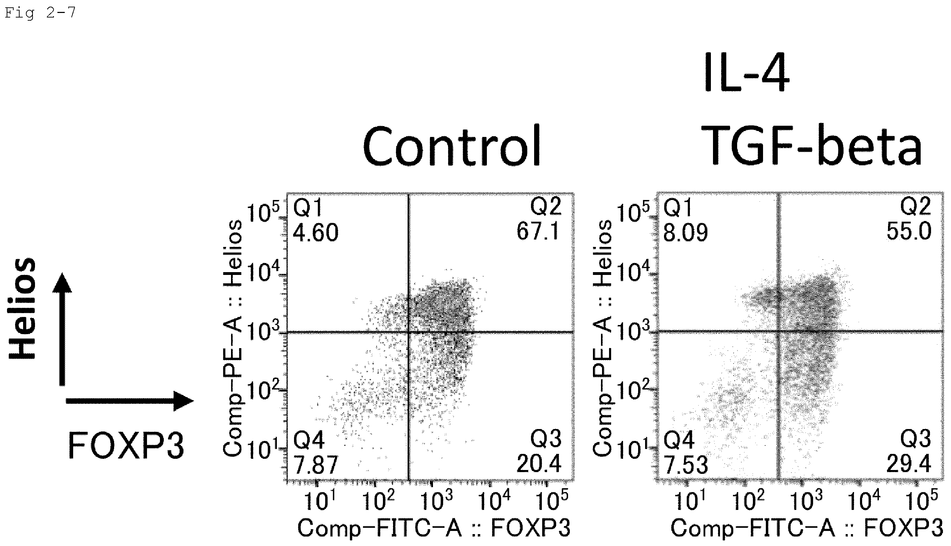

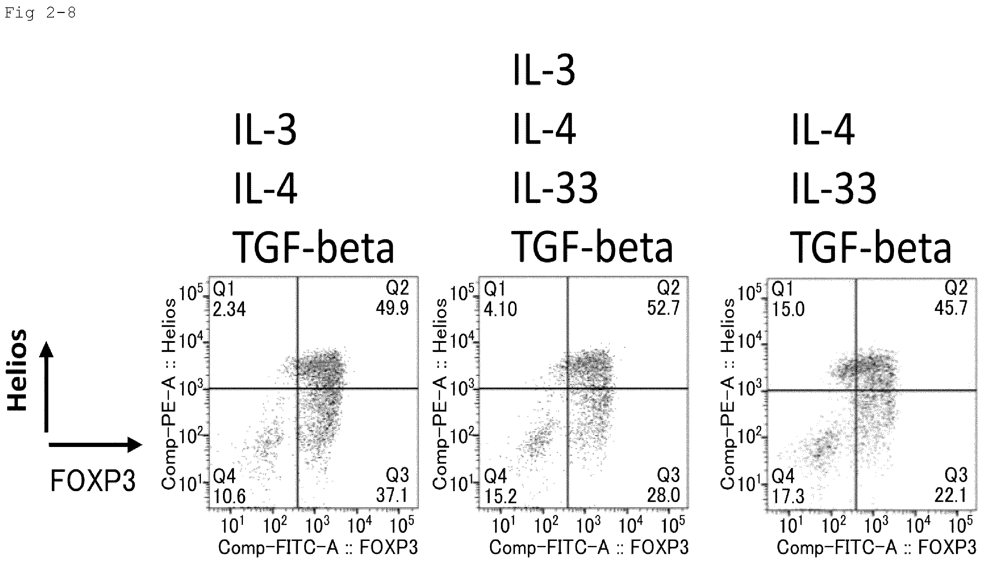

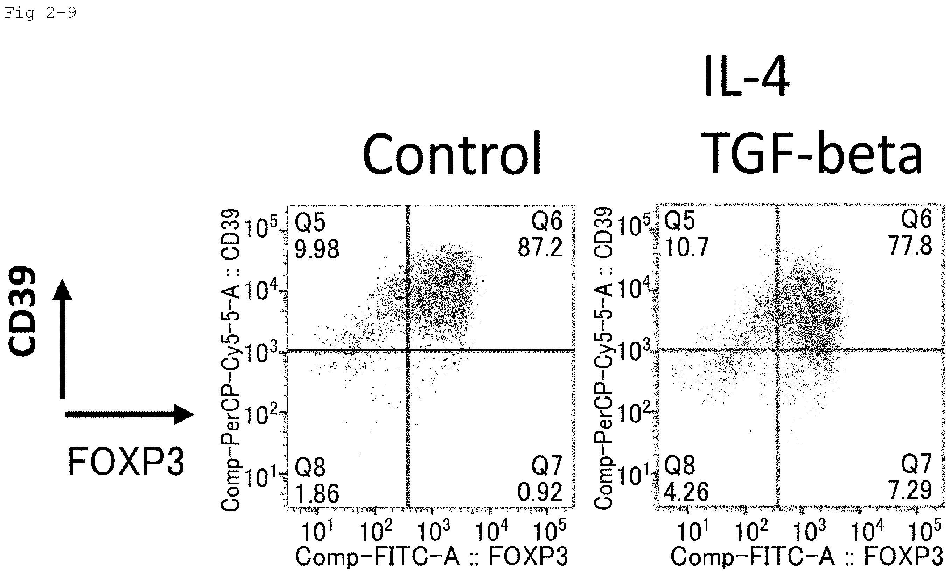

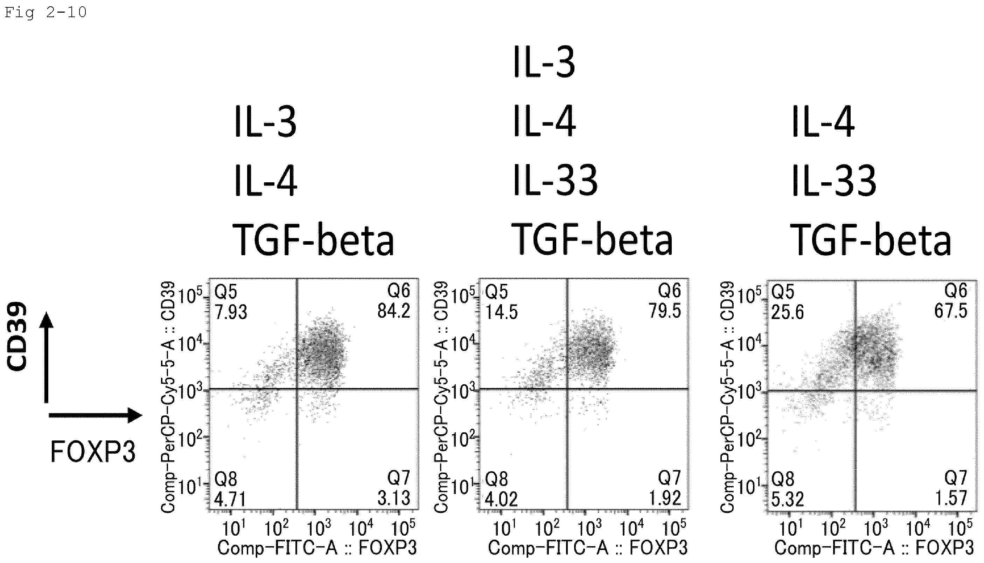

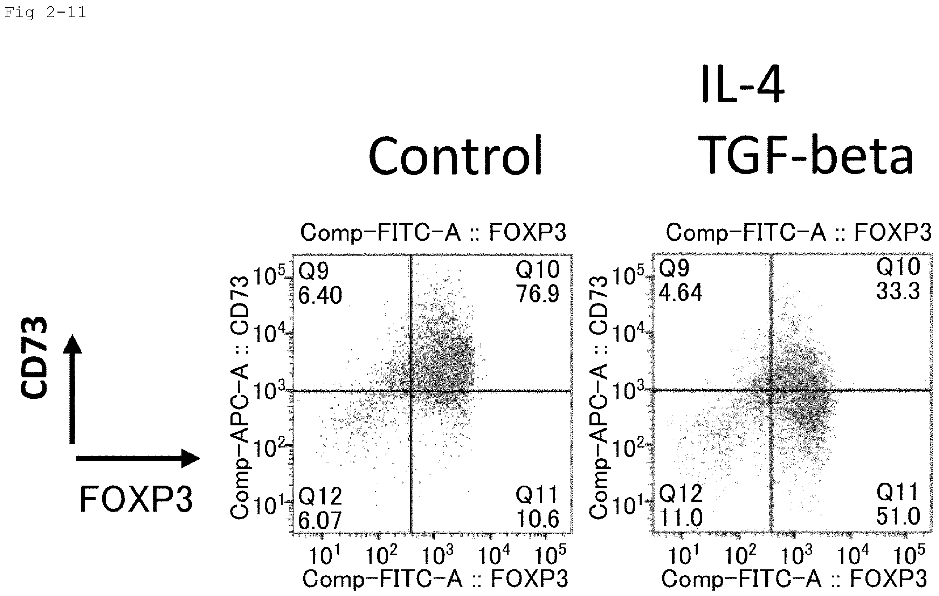

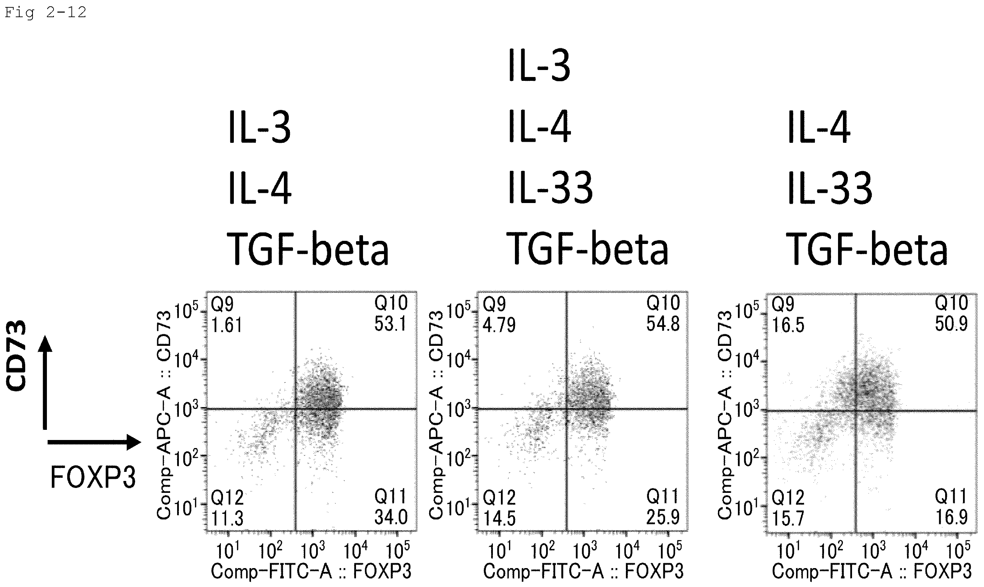

Fig. 2 shows the results of examining the expression levels of proteins expressed in Tregs expanded by culture in Example 1. The dot plotting in all of the graphs represents a population of CD3⁺CD4⁺CD8⁻. The values in the first row of the graphs show the percentage of Tregs (CD3⁺CD4⁺CD8⁻CD25⁺FOXP3⁺) in individual cell populations.

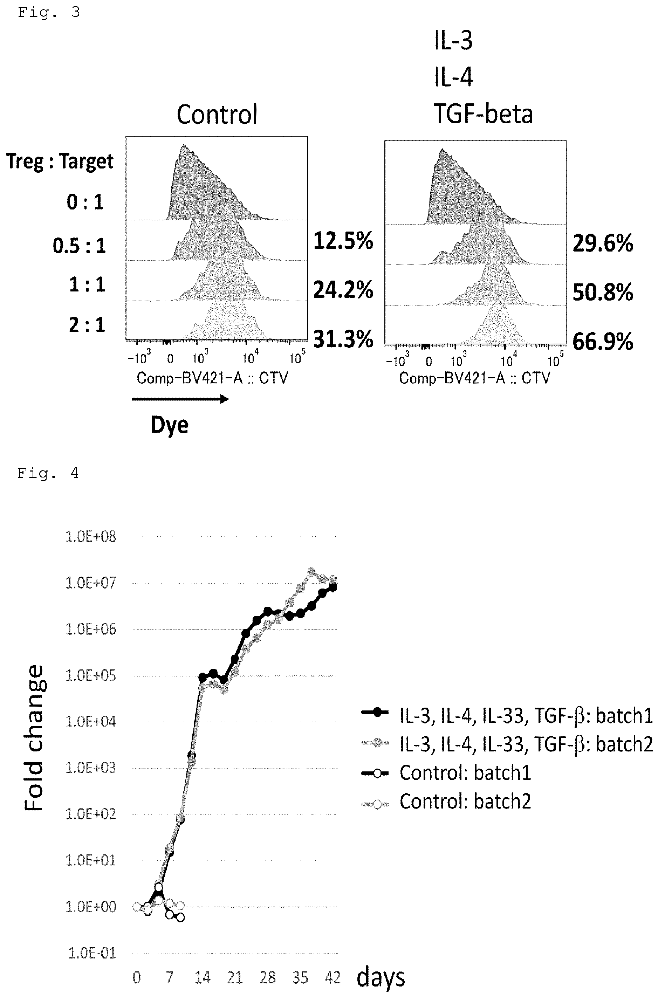

Fig. 3 shows the results of analysis by flow cytometry after co-culture of Tregs expanded by culture in Example 1 with allogeneic T cells derived from human PBMCs. The values by percentage shown in the figures indicate the inhibition rate of cell division in target cells. "Treg: Target" indicates the ratio of Tregs to human T cells in cell count.
Fig. 4 shows the changes in cell count over time when cells that have undergone differentiation induction from iPS cells to CD4⁺ T cells were repeatedly expanded by culture in a medium with or without IL-4, TGF-β1, IL-3, and IL-33.

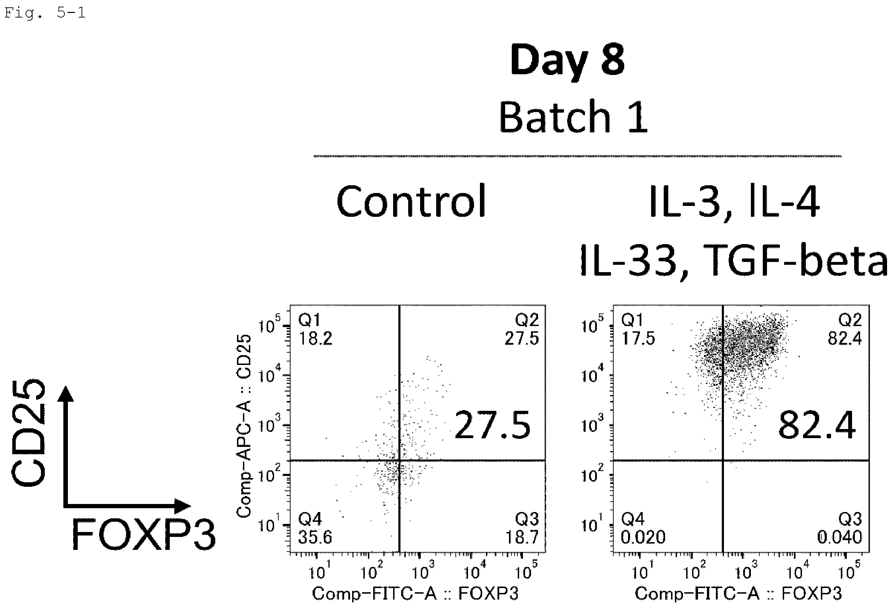

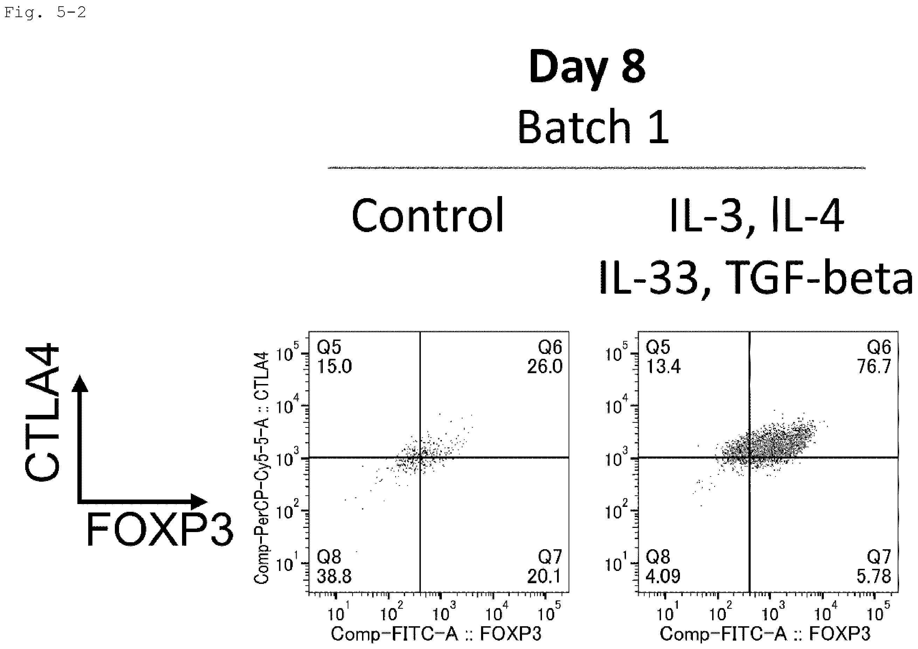

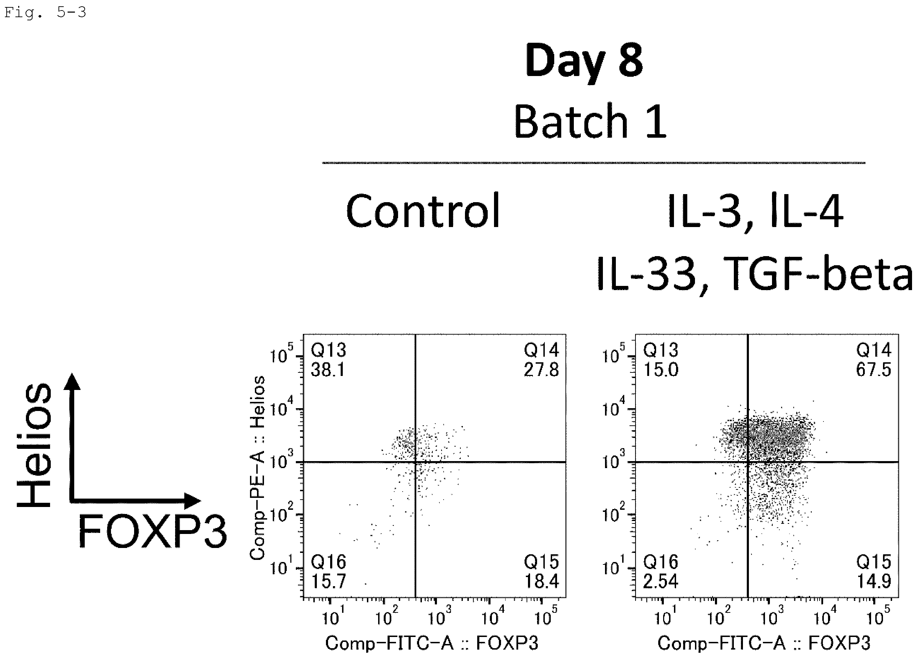

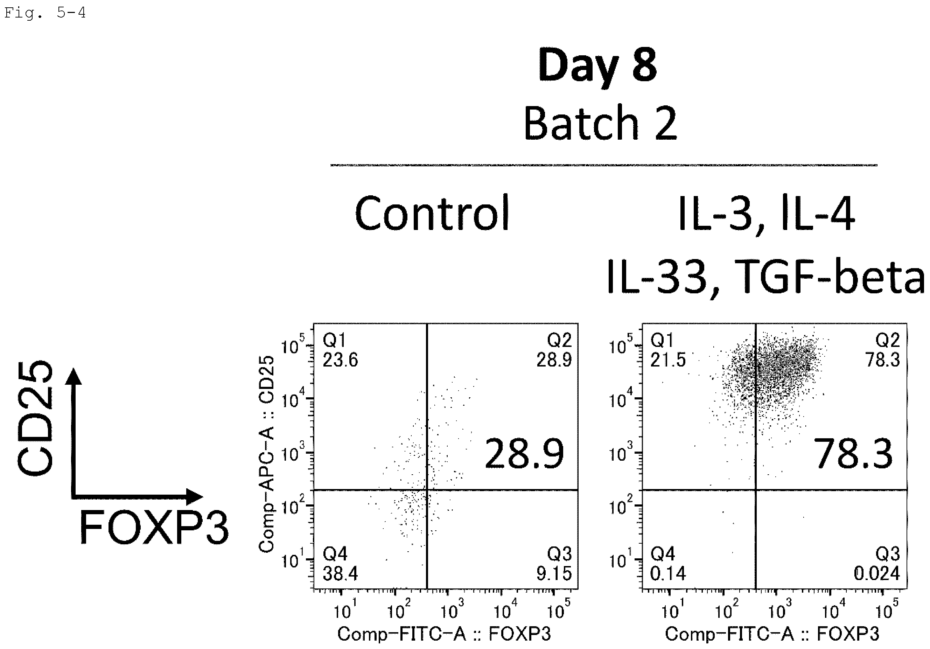

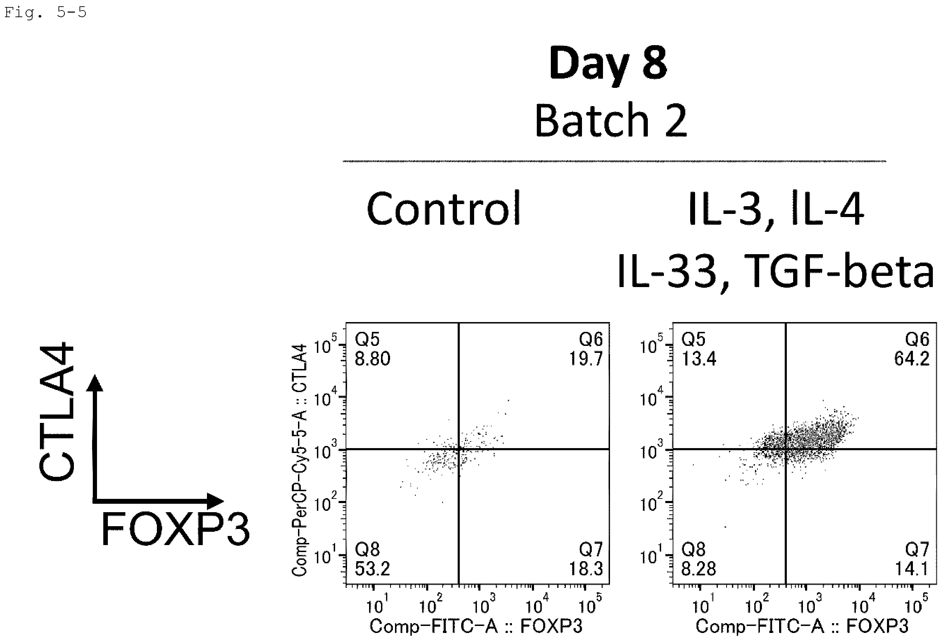

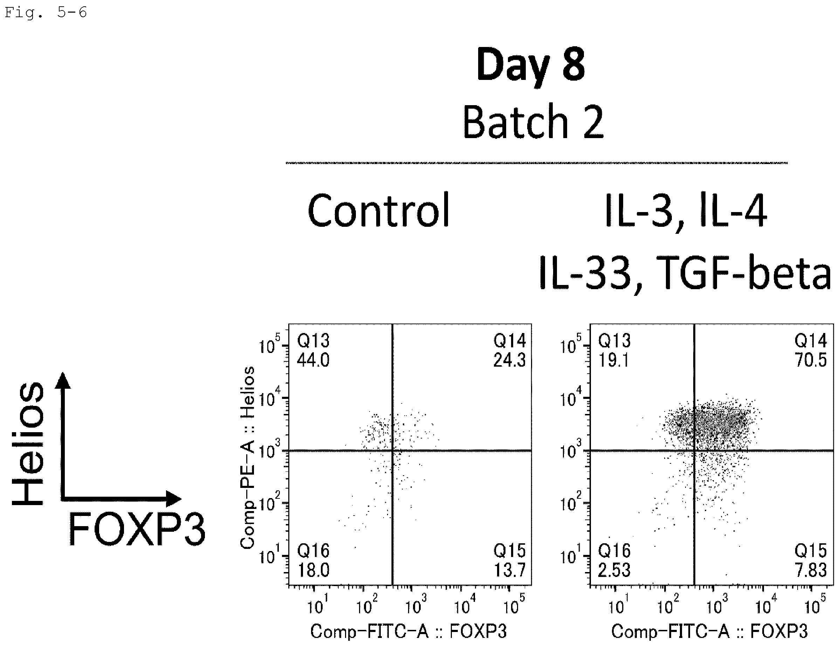

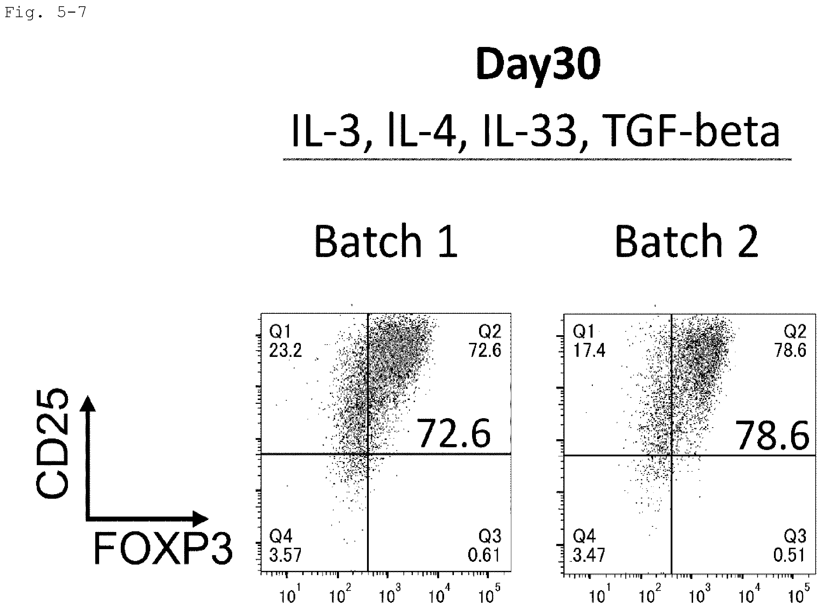

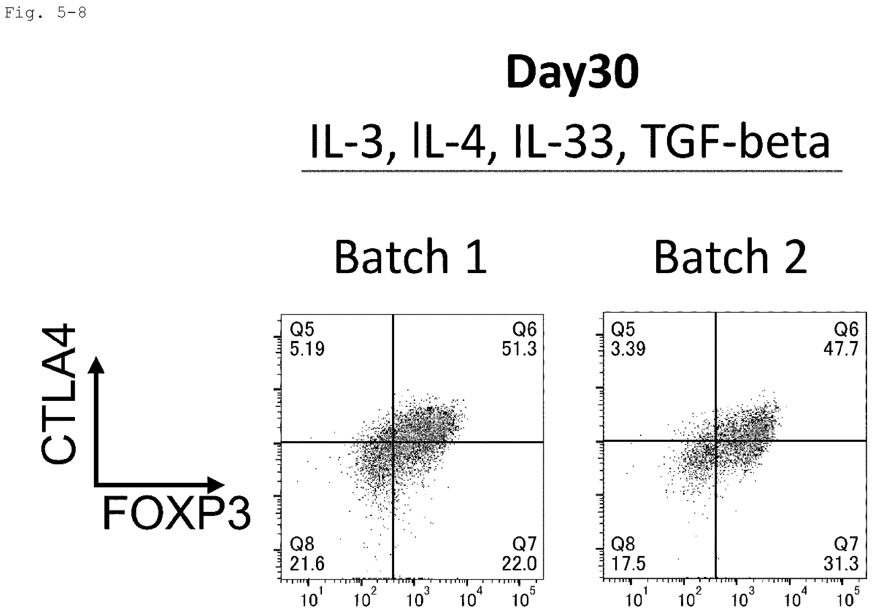

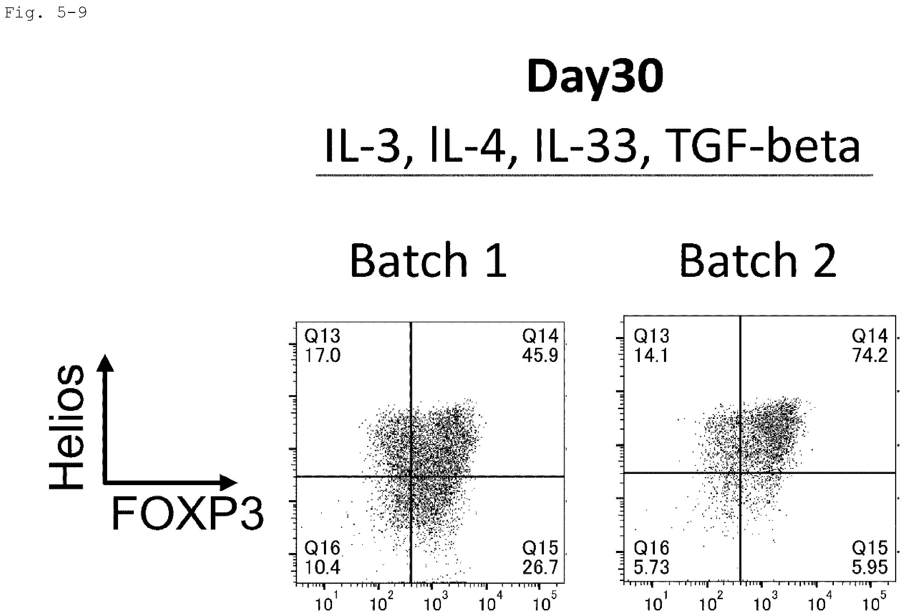

Fig. 5 shows the results of examining the expression levels of proteins expressed in Tregs using the cell population expanded by culture in Example 4. The dot plotting in all of the graphs represents a population of CD3⁺CD4⁺CD8⁻. The values in the graphs show the percentage of Tregs (CD3⁺CD4⁺CD8⁻CD25⁺FOXP3⁺) in individual cell populations.

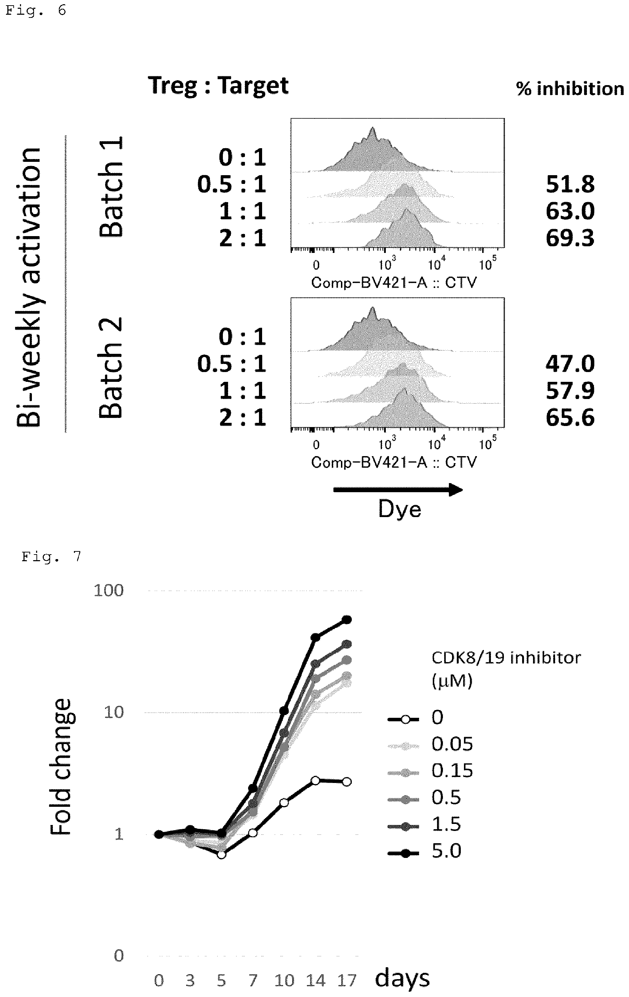

Fig. 6 shows the results of analysis by flow cytometry after co-culture of the cell population expanded by culture in Example 4 with allogeneic T cells derived from human PBMCs. The values by percentage shown in the figures indicate the inhibition rate of cell division in target cells. "Treg: Target" indicates the ratio of Tregs to human T cells in cell count.
Fig. 7 shows changes in cell count over time when cells that have undergone differentiation induction from iPS cells into CD4⁺ T cells were expanded by culture in a medium containing IL-4, TGF-β1, IL-3, IL-33, and a CDK8/19 inhibitor.

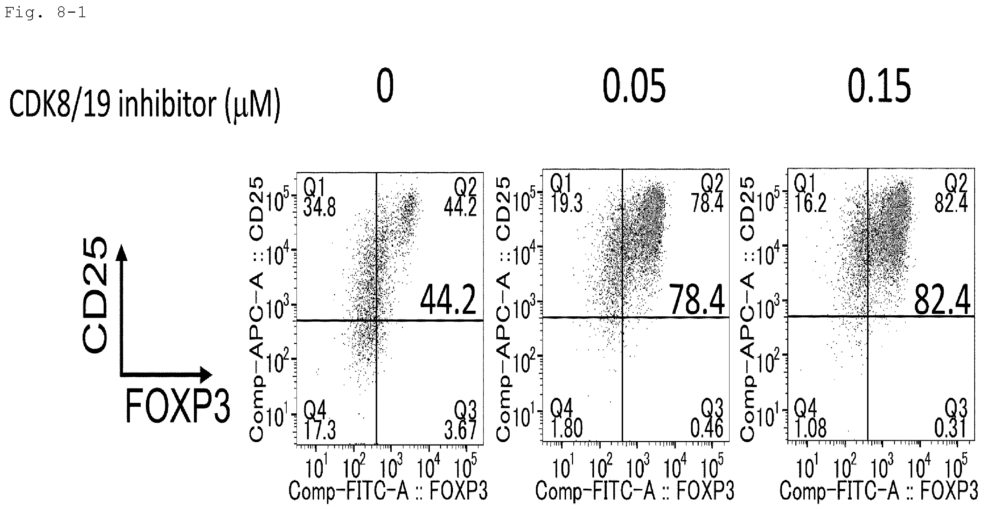

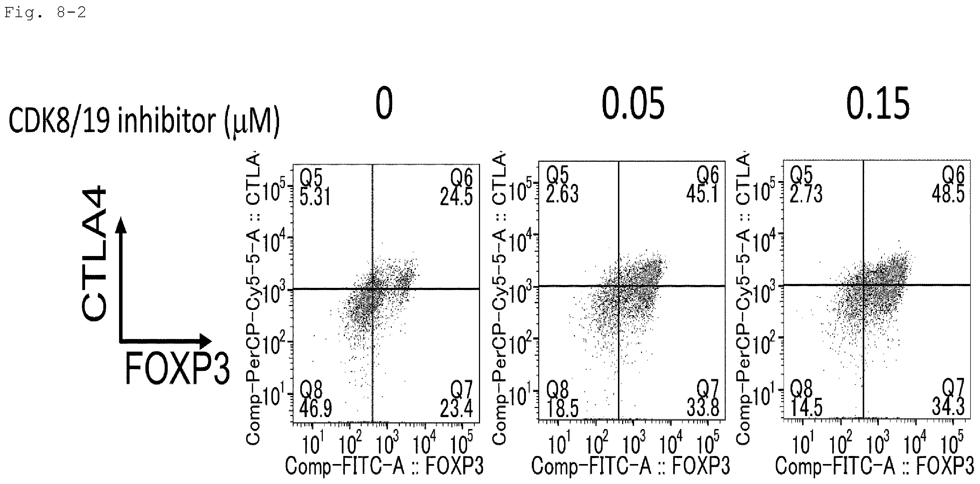

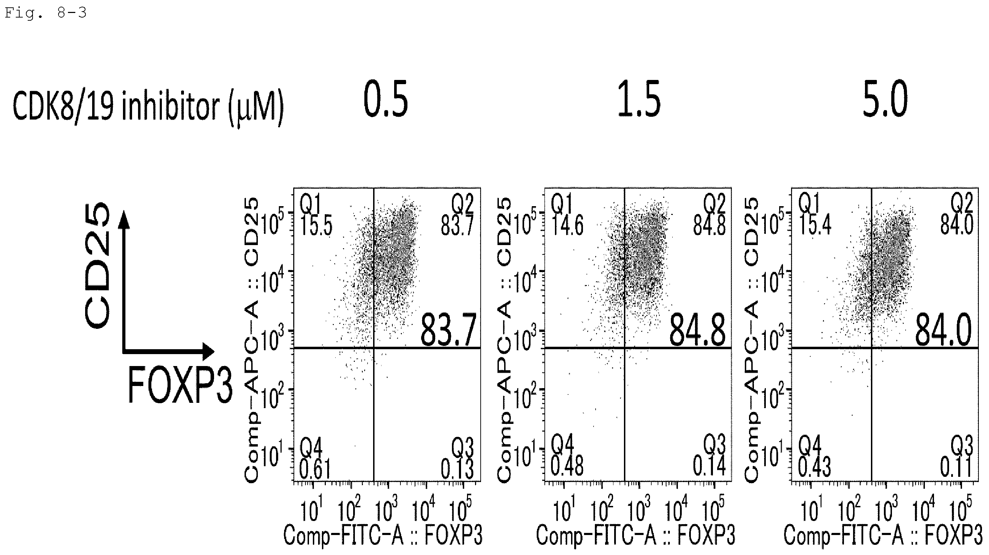

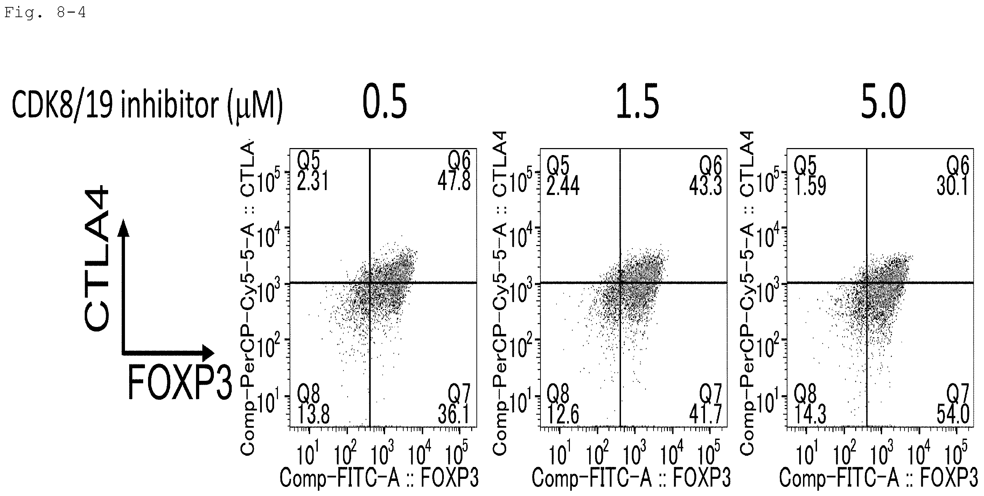

Fig. 8 shows the results of examining the expression levels of proteins expressed in Tregs using the cell population expanded by culture in Example 7. The dot plotting in all of the graphs represents a population of CD3⁺CD4⁺CD8⁻. The values in the graphs show the percentage of Tregs (CD3⁺CD4⁺CD8⁻CD25⁺FOXP3⁺) in individual cell populations.

### Description of Embodiments

Hereinafter, embodiments of the present invention will be described in detail.

The term "comprise(s)" or "comprising" means inclusion of the element(s) following the word without limitations thereto. Accordingly, this suggests inclusion of the element(s) following the word, but does not suggest exclusion of any other element. The phrase "consist(s) of" or "consisting of" means inclusion of all the element(s) following the phrase and limitation thereto. Accordingly, the phrase "consist(s) of" or "consisting of" indicates that the enumerated element(s) are required or essential and that substantially no other elements exist. The phrase "consist(s) essentially of" or "consisting essentially of" means inclusion of any element following the phrase and limitation of other elements to those that do not affect the activity or effect of the enumerated element(s) specified in the present disclosure. Accordingly, the phrase "consist(s) essentially of" or "consisting essentially of" indicates that the enumerated element(s) are required or essential, but other elements are optional and may exist or not exist depending on whether they affect the activity or effect of the enumerated element (s).

In the present specification, the term "culture" refers to maintenance or/and proliferation of cells in an in vitro environment. The term "culturing" refers to maintaining or/and proliferating cells outside tissue or outside the body, for example, in a cell culture dish or flask.

In the present specification, the phrase "culturing a cell population in the presence of a substance" refers to, for example, culturing a cell population in a medium containing the substance. Examples of such culture include culture in a medium containing the substance alone or in a medium containing the substance, other differentiation-inducing factors, and the like. When the substance is added to the medium, it may be added directly to the medium, or may be dissolved in an appropriate solvent before use and then added to the medium. Further, culture can also be performed with the substance immobilized on a substrate and carrier surface for culture.

In the present specification, the term "positive (+)" means that a protein or gene is expressed in detectable amounts by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNAseq.

In the present specification, the term "negative (-)" means that the expression level of a protein or gene is less than the lower limit of detection by all or any of the known methods described above, or the degree of expression thereof is low. The lower limit of detection of protein or gene expression may vary depending on the method. The degree of protein or gene expression (low expression or high expression) can be determined by comparison with the result of control cells measured under the same conditions. For example, the degree of CD25 expression in a certain cell population can be determined by comparing the expression level of CD25 in the cell population with the expression level of CD25 in PBMC-derived Tconv (control: known to be low in CD25 expression) using flow cytometry; when expression equivalent to that of the control is observed, it can be determined as low expression, and when expression is higher than that of the control cells, it can be determined as high expression.

In the present specification, the term "marker" refers to a protein or its gene that is specifically expressed on the cell surface, in the cytoplasm, or in the nucleus of a given cell type. The marker is preferably a "cell surface marker." The "cell surface marker" refers to a protein expressed on the cell surface that can be labeled (stained) with fluorescent substances and that facilitates the detection, condensation, isolation, or the like of cells expressing the cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in the genome or as a protein by translation of the mRNA.

Such cell surface markers can be detected by immunological assays using antibodies specific for the cell surface markers, such as ELISA, immunostaining, and flow cytometry.

In the present specification, the term "expression" is defined as the transcription and/or translation of a specific nucleotide sequence driven by an intracellular promoter.

In the present specification, "pluripotent stem cells" refer to embryonic stem cells (ES cells) and cells with similar pluripotency, i.e., cells with the potential to differentiate into various tissues (endoderm, mesoderm, and ectoderm) of a living body. Examples of cells with the same pluripotency as that of ES cells include "induced pluripotent stem cells" (also referred to as "iPS cells" in the present specification).

In the present specification, "hematopoietic stem cells (HSCs)" are multipotent stem cells that can differentiate into blood cells. Hematopoietic stem cells are mainly present in the bone marrow in a human living body, and differentiate into white blood cells (neutrophils, eosinophils, basophils, lymphocytes, monocytes, and macrophages), red blood cells, platelets, mast cells, dendritic cells, and the like. In the present specification, hematopoietic stem cells (HSCs) can be positive to CD34 and negative to CD7 (CD34⁺/CD7⁻). In the present specification, "/" used in the phrase "CD34⁺/CD7⁻" etc. means "and."

In the present specification, "hematopoietic progenitor cells" (HPCs) are cells that have the ability to differentiate into blood cells but do not have the ability to self-renew as much as stem cells. Hematopoietic progenitor cells are mainly present in the bone marrow in a human living body. In the present specification, hematopoietic progenitor cells (HPCs) can be positive to CD34 and positive to CD43 (CD34⁺/CD43⁺). Further, HPCs can be positive to CD24, CD62L, CD90, CD143, CD263, Notch3, CD32, CD39, CD49a, CD164, CD317, CD200, CD218a, CD7, CD144, CD56, CD226, CD262, and CD325, and negative to CD49f, CD51, CD102, CD42b, CD61, CD62P, CD69, CD102, and CD156c, as described in WO2018/199186.

In the present specification, "hemogenic endothelial cells (HECs)" are cells that express CD34 but do not express CD43, CD184, and CD73 (CD34⁺/CD43⁻/CD184⁻/CD73⁻) (CD34⁺/CD43⁻/CD184⁻/CD73⁻ cells do not express CD7, and CD34⁺/CD43⁻/CD184⁻/CD73⁻ cells are thus also referred to as "CD34⁺/CD7⁻/CD43⁻/CD184⁻/CD73⁻ cells") .

In the present specification, "progenitor T cells (proT)" refer to hematopoietic cells produced in the process of differentiation from hematopoietic stem cells into CD3-positive T cells in a human living body, and are positive to CD34 and CD7 (CD34⁺/CD7⁺ cells). Further, in the present specification, proT can be negative to CD43, CD1a, and/or CD116.

In the present specification, "mesodermal progenitor cells" refer to, for example, cells that express at least one marker gene selected from the group consisting of T (synonymous with Brachyury), KDR, FOXF1, FLK1, BMP4, MOX1, and SDF1. Mesodermal progenitor cells are not distinguished from mesodermal cells, and cells with weak expression of the above marker genes may be referred to as "mesodermal progenitor cells."

In the present specification, "regulatory T cells" refer to T cells that have the ability to inhibit the activation of effector T cells when stimulated via T cell receptors and that are responsible for the suppression of immune responses (immune tolerance). Regulatory T cells are generally CD25⁺/FOXP3⁺ cells or CD25⁺/CD127⁻ cells, and CD4⁺ or CD8⁺ cells are present therein. In the present invention, the regulatory T cells may be CD4⁺ cells (i.e., CD4⁺/CD25⁺/FOXP3⁺ or CD4⁺/CD25⁺/CD127⁻) or CD8⁺ cells (i.e., CD8⁺/CD25⁺/FOXP3⁺ or CD8⁺/CD25⁺/CD127⁻), and more preferably CD4⁺ cells. Further, the transcription factor FOXP3 is known as a master regulator of CD4⁺/CD25⁺ regulatory T cells. Furthermore, regulatory T cells may be positive for Helios, CTLA4 (cytotoxic T lymphocyte (associated) antigen 4) (CD152), CD39, and CD73, which are known indicators of the suppressive function of regulatory T cells. Helios is a member of the Ikaros transcription factor family, and has been shown to bind to the FOXP3 promoter and increase FOXP3 expression. CTLA4 is an immune checkpoint protein. CTLA4 expressed on the surface of T cells binds to CD80 or CD86 on the surface of antigen-presenting cells to inhibit T cell activation. It is also a known mechanism that CD39 and CD73 hydrolyze ATP and AMP respectively, producing adenosine as the final product, and adenosine acting on T cells suppresses T cell activation.

In the present specification, "CD4⁺ T cells" refer to, among T cells, those with surface antigens CD4 being positive. Preferably, CD4⁺ T cells are negative to CD8 (CD4⁺/CD8⁻, CD4 single positive (SP)); in this case, since T cells can be identified by surface antigens CD3 and CD45 being positive, CD4⁺ T cells can be identified as cells that are negative to CD8 and positive to CD4, CD3, and CD45 (CD4⁺/CD8⁻/CD3⁺/CD45⁺ cells). Examples of CD4⁺ T cells include helper T cells and regulatory T cells. The CD4⁺ T cells in the present specification are preferably CD4⁺/CD25⁻/FOXP3⁻cells or CD4⁺/CD25⁺/FOXP3⁺ cells.

In the present specification, "CD8⁺ T cells" refer to, among T cells, those with surface antigens CD8 being positive. Preferably, CD8⁺ T cells are negative to CD4 (CD4⁻/CD8⁺, CD8 single positive (SP)); in this case, since T cells can be identified by surface antigens CD3 and CD45 being positive, CD8⁺ T cells can be identified as cells that are negative to CD4 and positive to CD8, CD3, and CD45 (CD4⁻/CD8⁺/CD3⁺/CD45⁺ cells). Examples of CD8⁺ T cells include cytotoxic T cells and regulatory T cells. The CD8⁺ T cells in the present specification are preferably CD8⁺/CD25⁻/FOXP3⁻cells or CD8⁺/CD25⁺/FOXP3⁺ cells.

In the present specification, "stromal cells" are cells constituting the connective tissue that supports parenchymal cells in a living tissue, and particularly refer to stromal cells capable of producing an ATO (artificial thymic organoid) that produces T cells, typically, stromal cells contained in hematopoietic tissues such as bone marrow or thymus, and examples thereof include fibroblasts, blood cells, mesenchymal stem cells, endothelial cells, smooth muscle cells, epithelial cells, and tissue stem cells. The stromal cells may be established cells such as MS-5, OP9, and S17, which are mouse bone marrow cell strains, or HS-5 and HS-27a, which are human stromal cell strains, or may be primary cells collected from humans or the like, and cells differentiated from pluripotent stem cells (iPS cells etc.) of humans or the like; in the case of cells induced to differentiate from pluripotent stem cells of humans or the like, stromal cells induced to differentiate from human iPS cells (particularly, fibroblasts induced to differentiate from human iPS cells) are preferred.

In the present specification, "CD4CD8 double-positive T cells" refer to, among T cells, those with surface antigens CD4 and CD8 being both positive (CD8⁺/CD4⁺); since T cells can be identified by surface antigens CD3 and CD45 being positive, CD4CD8 double-positive T cells can be identified as cells that are positive to CD4, CD8, CD3, and CD45 (CD8⁺/CD4⁺/CD3⁺/CD45⁺ cells) .

In the present specification, "CD4CD8 double-negative T cells" refer to, among T cells, those with surface antigens CD4 and CD8 being both negative (CD8⁻/CD4⁻); since T cells can be identified by surface antigens CD3 and CD45 being positive, CD4CD8 double-negative T cells can be identified as cells that are negative to CD4 and CD8 and positive to CD3 and CD45 (CD8⁻/CD4⁻/CD3⁺/CD45⁺ cells).

In the present specification, "cell population" means two or more cells of the same type or different types. The "cell population" also means a mass of cells of the same type or different types.

In the present specification, the phrase "regulatory T cells have proliferated" or "proliferating regulatory T cells" indicates an increase in the number (absolute number) of regulatory T cells in a cell population compared to that before culture or to a control such as cells obtained without performing the present invention. For example, the phrases mean an increase of at least 1%, 5%, 10%, 20%, 30%, 40%, 50%, 60%, 70%, 80%, 90%, 100%, 120%, 150%, 200%, 300%, 400%, 500%, 600%, 700%, 800%, 900%, or 1000% compared to the number of the cells before culture or that of a control. In specific embodiments of the present invention, the cell population to be produced according to the present invention is produced so as to increase the number (absolute number) of regulatory T cells by at least 1%, 5%, 10%, 20%, 30%, 40%, 50%, 60%, 70%, 80%, 90%, 100%, 120%, 150%, 200%, 300%, 400%, 500%, 600%, 700%, 800%, 900%, or 1000%, as compared to the number of the cells before culture or that of a control.

The various cells used in the present invention are preferably cells certified by Good Manufacturing Practice (GMP), from the viewpoint of therapeutic applications.

Examples of pluripotent stem cells include embryonic stem cells (ES cells), induced pluripotent stem cells (iPS cells), embryonal carcinoma cells (EC cells), embryonic germ stem cells (EG cells), and Muse cells; and preferably iPS cells (more preferably human iPS cells). When the pluripotent stem cells are ES cells or any cells derived from the human embryo, the cells may be produced by destroying the embryo or without destroying the embryo, and preferably cells produced without destroying the embryo.

Regarding "ES cells," various mouse ES cell strains established by Ingenious Targeting Laboratory, RIKEN, etc. can be used as mouse ES cells, and various human ES cell strains established by the University of Wisconsin, NIH, RIKEN, Kyoto University, the National Center for Child Health and Development, Cellartis, etc. can be used as human ES cells. Examples of usable human ES cell strains include CHB-1 to CHB-12 strains, RUES1 strain, RUES2 strain, HUES1 to HUES28 strains, etc. furnished by ESI Bio; H1 strain, H9 strain, etc. furnished by WiCell Research; and KhES-1 strain, KhES-2 strain, KhES-3 strain, KhES-4 strain, KhES-5 strain, SSES1 strain, SSES2 strain, SSES3 strain, etc. furnished by RIKEN.

"Induced pluripotent stem cells" refer to cells obtained by reprogramming mammalian somatic cells or undifferentiated stem cells by introduction with specific factors (nuclear reprogramming factors). Currently, there are many different types of induced pluripotent stem cells. Examples of usable iPS cells include iPS cells established by Yamanaka et al. by introducing four factors, Oct3/4, Sox2, Klf4, and c-Myc, into mouse fibroblasts (Takahashi K., Yamanaka S., Cell, (2006) 126: 663-676), as well as human cell-derived iPS cells established by introducing the same four factors into human fibroblasts (Takahashi K., Yamanaka S., et al., Cell (2007) 131: 861-872), Nanog-iPS cells established by introducing the above four factors, followed by screening using the expression of Nanog as an index (Okita K., Ichisaka T., and Yamanaka S., Nature (2007) 448, 313-317), iPS cells produced by a method that does not include c-Myc (Nakagawa M., Yamanaka S., et al., Nature Biotechnology (2008) 26, 101-106), and iPS cells established by introducing six factors by a virus-free method (Okita K. et al., Nat. Methods 2011 May; 8(5): 409-12, Okita K. et al., Stem Cells. 31(3): 458-66). Other examples of usable iPS cells include induced pluripotent stem cells produced and established by Thomson et al. by introducing four factors, OCT3/4, SOX2, NANOG, and LIN28 (Yu J., Thomson J. A. et al., Science (2007) 318: 1917-1920), induced pluripotent stem cells produced by Daley et al. (Park I-H., Daley G. Q. et al., Nature (2007) 451: 141-146), induced pluripotent stem cells produced by Sakurada et al. (JP2008-307007A), and the like.

Other usable examples are any of the induced pluripotent stem cells known in the art and described in all of the published articles (e.g., Shi Y., Ding S., et al., Cell Stem Cell (2008) Vol 3, Issue 5, 568-574; Kim J. B., Scholer H. R., et al., Nature (2008) 454, 646-650; Huangfu D., Melton, D. A., et al., Nature Biotechnology (2008) 26, No 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852).

As induced pluripotent stem cell strains, various iPS cell strains established by NIH, RIKEN, Kyoto University, etc. can be used. Examples of human iPS cell strains include HiPS-RIKEN-1A strain, HiPS-RIKEN-2A strain, HiPS-RIKEN-12A strain, and Nips-B2 strain (RIKEN); Ff-I01s04 strain, QHJI strain, RWMH strain, DRXT strain, RJWI strain, YZWJ strain, ILCL strain, GLKV strain, 253G1 strain, 201B7 strain, 409B2 strain, 454E2 strain, 606A1 strain, 610B1 strain, and 648A1 strain (Kyoto University); and the like.

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog, and the nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid, and specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing a cell population in which regulatory T cells have proliferated of the present invention (also referred to simply as "the production method of the present invention" in the present specification) comprises the following step:
1) culturing a cell population containing CD4⁺ T cells derived from pluripotent stem cells in the presence of IL-4 and a TGF-βR agonist.

Step (1) can also be performed in the presence of IL-3 and/or IL-33, in addition to IL-4 and a TGF-βR agonist. In other words, step (1) can also be performed in the presence of IL-4, a TGF-βR agonist, and IL-3, in the presence of IL-4, a TGF-βR agonist, and IL-33, or in the presence of IL-4, a TGF-βR agonist, IL-3, and IL-33.

Furthermore, step (1) can be performed in the presence of at least one selected from the group consisting of IL-2, a TNFR2 agonist, and an mTOR inhibitor, in particular, in the presence of IL-2, a TNFR2 agonist, and an mTOR inhibitor, in addition to the components above.

Additionally, step (1) can be performed in the presence of a CDK8 and/or CDK19 inhibitor in addition to the components above.

IL (interleukin)-4, IL (interleukin)-3, IL (interleukin)-2, and IL (interleukin)-33 are preferably derived from mammals, especially humans. IL-4, IL-3, IL-2, and IL-33 may also be natural substances isolated and purified from mammals (particularly humans), or those artificially produced through genetic engineering techniques (which may include amino acid substitutions, deletions, insertions, and/or additions).

The TGF-βR (transforming growth factor-β receptor) agonist is defined as a substance that can bind to TGF-βR and activate TGF-βR signaling. TGF-βR agonists include factors that belong to the TGF-β superfamily, which includes the TGF-β family, activin family, and BMP family (bone morphogenetic protein). Examples of such factors that belong to the TGF-β superfamily include TGF-β (TGF-β1, TGF-β2, and TGF-β3), activin A, activin B, GDF-8, and GDF-11. The TGF-βR agonist is preferably TGF-β, and more preferably TGF-β1. The TGF-βR agonist can be used alone or in a combination of two or more types.

The TNFR2 (tumor necrosis factor receptor-2) agonist is defined as a substance that can bind to TNFR2 and activate TNFR2 signaling, such as antibodies (e.g., anti-TNFR2 antibodies), peptides, low-molecular compounds, and proteins. Examples of TNFR2 agonist antibodies include monoclonal antibodies that bind to TNFR2, such as clone MR2-1 (Hycult Biotech) and clone MAB2261 (R&D Systems). The TNFR2 agonist can also be a TNF-α mutein that binds only to TNFR2 as an agonist. The TNFR2 agonist can be used alone or in a combination of two or more types.

The TNFR2 agonist is preferably a TNFR2 agonist antibody. The antibody may be its functional fragment, and examples of functional fragments include Fd, Fv, Fab, F(ab'), F(ab)₂, F(ab')₂, single-chain Fv (scFv), diabody, triabody, tetrabody, and minibody. The antibody can be derived from animals such as mice, rats, cattle, rabbits, goats, sheep, and guinea pigs. The isotype of the antibody is not particularly limited, and examples of isotypes include IgG (IgG1, IgG2, IgG3, and IgG4), IgA, IgD, IgE, and IgM. The antibody may also be either a monoclonal antibody or a polyclonal antibody, with a monoclonal antibody being preferred; the antibody may also be a humanized antibody, a chimeric antibody, or a multi-specific antibody (e.g., bispecific antibody). An antibody can be produced according to a known method, for example, by constructing an expression vector containing a nucleic acid encoding the antibody and culturing a transformant having the nucleic acid introduced, or by culturing a hybridoma that produces the antibody.

The mTOR (mechanistic target of rapamycin) inhibitor is defined as a substance that inhibits the function of mTOR, which includes substances that inhibit the function of mTOR itself, and substances that inhibit the function of mTOR complexes, namely mTOR complex 1 (mTORC1) and mTOR complex 2 (mTORC2). Examples of mTOR inhibitors include rapamycin, derivatives of rapamycin, everolimus, temsirolimus, ridaforolimus, sirolimus, KU0063794, AZD805, AZD8055, WYE-354, WAY-600, WYE-687, Pp121, Pp242, dactolisib, sapanisertib, omipalisib, vistusertib, torin 1, torin 2, and others. Other mTOR inhibitors include siRNA, shRNA, dsRNA, miRNA, antisense nucleic acids, and expression vectors expressing these, targeting the gene encoding mTOR and/or its transcription products, as well as siRNA, shRNA, dsRNA, miRNA, antisense nucleic acids, and expression vectors expressing these, targeting the gene encoding the enzyme that phosphorylates and activates mTOR and/or its transcription products. The mTOR inhibitor is preferably rapamycin or its derivatives, with rapamycin being more preferred. The mTOR inhibitor can be used alone or in a combination of two or more types.

The CDK8 (cyclin-dependent kinase 8) and/or CDK19 inhibitor (which may be referred to as a "CDK8/19 inhibitor" in the present specification) is defined as a substance that inhibits the function of CDK8 and/or CDK19, particularly a substance that inhibits the kinase activity of CDK8 and/or CDK19, and may be an inhibitor of both CDK8 and CDK19 or may be an inhibitor of either one. Examples of the CDK8 and/or CDK19 inhibitor include 4-[1-(2-methyl-1H-benzimidazol-5-yl)-1H-imidazo[4,5-c]pyridin-2-yl]-1,2,5-oxadiazole-3-amine (AS2863619) (hereinafter also referred to as "compound 1"); 3-{1-[1-(4-methoxyphenyl)piperidin-4-yl]-4-methyl-1H-imidazo[4,5-c]pyridin-2-yl}pyrazine-2-amine (AS3334366) (hereinafter also referred to as "compound 2"); siRNA, shRNA, dsRNA, miRNA, and antisense nucleic acid for genes encoding CDK8 and/or CDK19 and/or transcripts thereof, and expression vectors expressing them; compounds described in US8598344, WO2013/116786, Proc. Natl. Acad. Sci. U.S.A. 109 13799-13804 (2012), WO2013/001310, WO2013/040153, WO2014/029726, WO2014063778, WO2014/072435, WO2014/090692, WO2014/106606, WO2014/123900, WO2014/154723, WO2014/194245, WO2015/049325, WO2015/100420, WO2015/144290, WO2015/159937, and WO2015/159938 (particularly, (2E)-N-(2-fluoro-4-(morpholin-4-yl methyl)phenyl)-3-(4-(1-methyl-1H-pyrazol-4-yl)pyridin-3-yl)acrylamide (hereinafter also referred to as "compound 3") and (2E)-3-(4-(1-cyclopropyl-1H-pyrazol-4-yl)pyridin-3-yl)-N-(1-methyl-1H-indol-5-yl)acrylamide (hereinafter also referred to as "compound 4")); compounds described in WO2016/009076 and WO2018/159805 (particularly, 8-(2,4-difluorophenoxy)-1-methyl-4,5-dihydro-1H-thieno[3,4-g]indazol-6-carboxamide (hereinafter also referred to as "compound 5"), 4-((4-fluorophenyl)sulfonyl)-3-(2-(imidazo[1,2-b]pyridazin-6-yl sulfanyl)ethyl)-3,4-dihydroquinoxalin-2(1H)-one (hereinafter also referred to as "compound 6"), 2-(benzylamino)-4-(1H-pyrrolo[2,3-b]pyridin-3-yl)benzamide (hereinafter also referred to as "compound 7"), 3-(3-(benzyloxy)phenyl)-1H-pyrrolo[2,3-b]pyridin (hereinafter also referred to as "compound 8"), 4-(4-(2,3-dihydro-1,4-benzodioxin-6-yl)-1H-pyrazol-3-yl)benzen-1,3-diol (particularly, trifluoroacetic acid salt thereof) (hereinafter also referred to as "compound 9"), N-butyl-8-(4-methoxyphenyl)-1,6-naphthyridin-2-carboxamide, and 8-(4-methylphenyl)-N,N-dipropyl-1,6-naphthyridin-2-carboxamide (particularly, trifluoroacetic acid salt thereof) (hereinafter also referred to as "compound 10")); compounds described in WO2022/107877 (particularly, diethyl (E)-(4-(3-(5-(4-fluorophenyl)-1-methyl-1H-pyrazol-4-yl)acrylamide)benzyl)phosphonate (hereinafter also referred to as "compound 11"), 2-(4-(4-(isoquinolin-4-yl)phenyl)-1H-pyrazol-1-yl)-N,N-dimethylacetamide (BI-1347), and 4-((2-(6-(4-methylpiperazin-1-carbonyl)naphthalen-2-yl)ethyl)amino)quinazolin-6-carbonitrile (Senexin B)); MSC2530818 (CAS No.: 1883423-59-3) and CCT251921 (CAS No.: 1607837-31-9), and the like. Preferred among these are compound 1, compound 2, compound 3, compound 4, compound 5, compound 6, compound 7, compound 8, compound 9, compound 10, compound 11, BI-1347, Senexin B, MSC2530818, CCT251921, and siRNA for genes encoding CDK8 and/or CDK19 and/or transcripts thereof; more preferred are compound 1, compound 2, and siRNA for genes encoding CDK8 and/or CDK19 and/or transcripts thereof; and further preferred is compound 1. The CDK8 and/or CDK19 inhibitor can be used singly or in a combination of two or more types thereof. In addition, a method for inhibiting CDK8 and/or CDK19 can also be performed by knocking out a gene encoding CDK8 and/or CDK19. As a method for knocking out such a gene, for example, a method known in the art such as genome editing using ZFN, TALEN, CRISPR/Cas system, or the like can be used.

Further, IL-2, IL-4, the TGF-βR agonist, IL-3, IL-33, the TNFR2 agonist, the mTOR inhibitor, and the CDK8 and/or CDK19 inhibitor can be used in free form or in salt form. Examples of salts include salts with inorganic bases, such as sodium salts, magnesium salts, potassium salts, calcium salts, and aluminum salts; salts with organic bases, such as methylamine salts, ethylamine salts, and ethanolamine salts; salts with basic amino acids, such as lysine, ornithine, and arginine; and ammonium salts. These salts may be acid addition salts, and specific examples of such salts include addition salts with mineral acids, such as hydrochloric acid, hydrobromic acid, hydroiodic acid, sulfuric acid, nitric acid, and phosphoric acid; organic acids, such as formic acid, acetic acid, propionic acid, oxalic acid, malonic acid, malic acid, tartaric acid, fumaric acid, succinic acid, lactic acid, maleic acid, citric acid, methanesulfonic acid, and ethanesulfonic acid; and acidic amino acids, such as aspartic acid and glutamic acid. The IL-2, IL-4, TGF-βR agonist, IL-3, IL-33, TNFR2 agonist, mTOR inhibitor, and CDK8 and/or CDK19 inhibitor also include their hydrates, solvates, crystalline polymorphs, and the like.

The concentration of IL-4 in the medium is not particularly limited, and can be, for example, 0.01 to 100 ng/mL, and preferably 10 to 100 ng/mL.

The concentration of the TGF-βR agonist in the medium is not particularly limited and is suitably adjusted according to the type of the TGF-βR agonist used etc. The concentration of the TGF-βR agonist is, for example, 0.1 to 100 ng/mL, and preferably 1 to 50 ng/mL.

The concentration of IL-3 in the medium is not particularly limited and can be, for example, 0.01 to 100 ng/mL, and preferably 10 to 100 ng/mL.

The concentration of IL-33 in the medium is not particularly limited and can be, for example, 0.1 to 100 ng/mL, and preferably 10 to 100 ng/mL.

The concentration of IL-2 in the medium is not particularly limited and can be, for example, 0.1 to 100 ng/mL, and preferably 10 to 100 ng/mL.

The concentration of the TNFR2 agonist in the medium is not particularly limited, and is suitably adjusted according to the type of TNFR2 agonist used, etc. The concentration of the TNFR2 agonist is, for example, 0.0001 to 100 µg/mL, and preferably 0.01 to 10 µg/mL.

The concentration of the mTOR inhibitor in the medium is not particularly limited, and is suitably adjusted according to the type of mTOR inhibitor used, etc. The concentration of the mTOR inhibitor is, for example, 1 to 100 nM, and preferably 10 to 100 nM.

The concentration of the CDK8 and/or CDK19 inhibitor in the medium is not particularly limited and is suitably adjusted according to the type of the CDK8 and/or CDK19 inhibitor used etc. The concentration of the CDK8 and/or CDK19 inhibitor is, for example, 0.01 to 300 µM, and preferably 0.05 to 150 µM.

The medium used in the culture of step (1) contains IL-4, a TGF-βR agonist (as well as IL-2, IL-3, IL-33, a TNFR2 agonist, an mTOR inhibitor, a CDK8 and/or CDK19 inhibitor, etc.) in a basal medium used for culturing animal cells. The basal medium is not particularly limited as long as it can be used for culturing animal cells, and examples thereof include AIM V, X-VIVO-15, NeuroBasal, EGM2, TeSR, BME, BGJb, CMRL 1066, Glasgow's MEM, Improved MEM Zinc Option, IMDM, 199 medium, Eagle's MEM, αMEM, DMEM, Ham, RPMI-1640, Fischer's medium, and the like. These media may be used singly or as a mixture of two or more types thereof.

The medium may contain serum or may be serum-free. The medium may further contain serum replacements (e.g., albumin, transferrin, Knockout Serum Replacement (KSR), fatty acid, insulin, collagen precursors, minor elements, 2-mercaptoethanol, 3'-thiolglycerol, ITS-supplement, B27 (trademark) supplement, etc.). Serum replacements can be used singly or in a combination of two or more types thereof.

The medium may further contain one or more substances of lipids, amino acids (non-essential amino acids etc.), L-glutamine, vitamins, growth factors, cytokines (IL-2 etc.), anti-CD3 antibodies, anti-CD30 antibodies, anti-CD28 antibodies, antibiotics, antioxidants, pyruvic acid, buffers, inorganic salts, and the like. As the medium, it is desirable to use a chemically defined medium that does not contain materials with unknown components, such as serum, so as to reduce the difference in medium among lots and prepare cells with stable quality.

The pH of the medium is generally 7.0 to 7.8 and preferably 7.2 to 7.6. In order to prevent contamination before use, the medium is preferably sterilized according to a method such as filtration, UV irradiation, heat sterilization, or radiation.

The culture is performed in the presence or absence of feeder cells. Since the production method of the present invention can stably produce regulatory T cells having uniform properties without containing substances whose components are unknown, it is desirable to carry out the production method in the absence of feeder cells.

The culture conditions of the production method of the present invention are not particularly limited, and the culture temperature is, for example, about 37 to 42°C, preferably about 37 to 39°C, the CO₂ concentration is, for example, 2% to 10%, preferably 2 to 5%, and the oxygen concentration is, for example, 1 to 20%, preferably 5 to 20%.

The culture period is also not particularly limited, and can be suitably determined by a person skilled in the art while monitoring the number of regulatory T cells, and is, for example, 7 days or more, preferably 14 days or more, more preferably 21 days or more, and even more preferably 28 days or more. The upper limit of the culture period is not particularly limited, and is, for example, 42 days or less, preferably 35 days or less, and more preferably 28 days or less. In the culture in the present invention, passage may be performed as many times as necessary to obtain the desired amount of regulatory T cells, or addition and replacement of medium may be performed. The culture in the present invention can be performed using a known CO₂ incubator. The culture container is not particularly limited, and can be suitably selected from plates, dishes, petri dishes, flasks, bags, bottles, tanks (culture tanks), bioreactors, and the like. Culture containers for use can be those coated with anti-CD3 antibodies.

In step (1), cells may be cultured by repeating the following procedure: cells are first cultured (for stimulation) by using an anti-CD3 antibody and an anti-CD28 antibody in the presence of IL-4 and a TGF-βR agonist (optionally further with IL-3, IL-33, IL-2, a TNFR2 agonist, a CDK8/19 inhibitor and/or an mTOR inhibitor), and then cultured in a medium without the anti-CD3 antibody and anti-CD28 antibody. The period for this repeated culture is, for example, 6 to 16 days, and preferably 7 to 14 days. Specifically, the following unit of culture is repeated: after cells are cultured (stimulated) in the presence of an anti-CD3 antibody and an anti-CD28 antibody for about the first 3 days, the cells are then cultured in a medium without such anti-CD3 antibody and anti-CD28 antibody for about 4 to 11 days. The number of culture repetitions is not particularly limited, and those skilled in the art would be able to determine it appropriately while monitoring the number of regulatory T cells, or other factors. For example, the number of culture repetitions can be 1 to 5 times. Additionally, in the step of adding an anti-CD3 antibody and an anti-CD28 antibody, an anti-CD30 antibody may be further added to perform culture.

Pluripotent stem cells and cells for producing pluripotent stem cells used in the present invention may be derived from humans, or may be derived from mammals other than humans (non-human mammals), and are preferably derived from humans. Examples of non-human mammals include mice, rats, hamsters, guinea pigs, rabbits, dogs, cats, pigs, cows, horses, sheep, and monkeys. Further, universalized iPS cells (having a specific HLA that does not evoke immune rejection in a large number of patients, or having HLA knocked out) can also be used.

The cell population containing CD4⁺ T cells used in the production method of the present invention is a cell population containing CD4⁺ T cells induced to differentiate from pluripotent stem cells (particularly iPS cells (especially human iPS cells)). The proportion (number of cells) of CD4⁺ T cells contained in the cell population containing CD4⁺ T cells is, for example, 5% or more, preferably 10% or more, and more preferably 30% or more. The upper limit is, for example, 100% or less.

In order to concentrate the obtained regulatory T cells, the production method of the present invention may further comprise separating the regulatory T cells. The separation of regulatory T cells can be performed according to a known method such as a method using flow cytometry or magnetic cell separation.

The production method of the present invention enables the production of a cell population containing regulatory T cells, in which the regulatory T cells have proliferated. When CD25⁺/FOXP3⁺ cells (regulatory T cells) are used as CD4⁺ T cells derived from pluripotent stem cells, the regulatory T cells can be expanded by culture according to the production method of the present invention. When CD25⁻/FOXP3⁻ cells, which are not regulatory T cells, are used as CD4⁺ T cells derived from pluripotent stem cells, the cells can be induced to differentiate into regulatory T cells to produce a cell population with a high proportion of regulatory T cells.

When CD25⁻/FOXP3⁻ cells are used as CD4⁺ T cells, the proportion (cell count) of regulatory T cells in the cell population containing regulatory T cells obtained according to the production method of the present invention is, for example, 10% or more, preferably 50% or more, and more preferably 80% or more, with the upper limit being, for example, 100% or less.

The production method of the present invention can also be applied to a cell population containing CD8⁺ T cells in addition to a cell population containing CD4⁺ T cells.

Additionally, in an embodiment of the present invention in which CD25⁺/FOXP3⁺ cells, which are regulatory T cells, are used as CD4⁺ T cells derived from pluripotent stem cells, the method for proliferating a cell population containing regulatory T cells comprises the following step:
(1A) culturing a cell population containing regulatory T cells derived from pluripotent stem cells in the presence of IL-4 and a TGF-βR agonist.

In an embodiment of the present invention in which CD25⁻/FOXP3⁻ cells, which are not regulatory T cells, are used as CD4⁺ T cells derived from pluripotent stem cells, the method for producing a cell population containing regulatory T cells comprises the following step:
(1B) culturing a cell population containing CD4⁺/CD25⁻/FOXP3⁻ T cells derived from pluripotent stem cells in the presence of IL-4 and a TGF-βR agonist.

Steps (1A) and (1B) can be performed in the same manner as step (1). The method for proliferating a cell population containing regulatory T cells and the method for producing a cell population containing regulatory T cells described above are encompassed by the production method of the present invention, and the explanation regarding the production method of the present invention applies to these methods.

The production method and the proliferation method of the present invention may further comprise (2) inducing differentiation of pluripotent stem cells into a cell population containing CD4⁺ T cells before step (1).

The differentiation of pluripotent stem cells into a cell population containing CD4⁺ T cells can be induced according to a known method. Examples of such methods include inducing differentiation of pluripotent stem cells into cells that can differentiate into CD4⁺ T cells, and then inducing differentiation of the cells that can differentiate into CD4⁺ T cells into a cell population containing CD4⁺ T cells. Examples of the cells that can differentiate into CD4⁺ T cells include CD34⁺ cells, mesodermal progenitor cells, CD4CD8 double-negative T cells, CD4CD8 double-positive T cells, and the like. CD34⁺ cells or mesodermal progenitor cells are preferred, hemogenic endothelial cells, hematopoietic stem cells, hematopoietic progenitor cells, progenitor T cells, or mesodermal progenitor cells are more preferred, and hemogenic endothelial cells are particularly preferred. The CD34⁺ cells are (CD34⁺) cells expressing CD34, and particularly are (CD34⁺/CD7⁻) cells expressing CD34 and not expressing CD7. Examples of CD34⁺ cells include hemogenic endothelial cells, hematopoietic stem cells, and hematopoietic progenitor cells.

The differentiation of pluripotent stem cells into cells that can differentiate into CD4⁺ T cells can be induced according to a known method (see, for example, WO2021/085576 etc.). For example, cells that can differentiate into CD4⁺ T cells can be produced according to a feeder-free method, and in particular, an "embryonic body method (EB method)" (see AE Grigoriadis et al. (2010). Blood, 115(14): 2769-2776) in which an embryonic body is formed without using feeder cells to produce hematopoietic progenitor cells is preferred.

Pluripotent stem cells can also be cultured on feeder cells to induce differentiation into hematopoietic stem cells and/or hematopoietic progenitor cells (see WO2011/096482 and WO2013/176197). The feeder cells are preferably stromal cells from the viewpoint of easily inducing differentiation into mesodermal lineage. The stromal cells are preferably OP9 cells, 10T1/2 cells (C3H10T1/2 cells), and the like from the viewpoint of facilitating differentiation into hematopoietic lineage.

The differentiation of pluripotent stem cells into CD34⁺ cells can be performed according to a known method. When pluripotent stem cells are iPS cells, for example, differentiation of hematopoietic progenitor cells can be induced by the methods described in WO2017/221975, WO2018/135646, and Cell Reports 2 (2012) 1722-1735 to produce CD34⁺ cells.

Examples of the method for inducing differentiation of cells that can differentiate into CD4⁺ T cells into a cell population containing CD4⁺ T cells include an artificial thymic organoid (ATO) method (see WO2017/075389 etc.). The ATO method comprises culturing a three-dimensional cell aggregate containing cells that can differentiate into CD4⁺ T cells and stromal cells expressing a Notch ligand, whereby differentiation into CD4⁺ T cells can be induced with high efficiency. The ATO method is a known method, and can be performed with reference to the description in WO2017/075389, WO2021/085576, and the like. Other methods include the differentiation induction method (e.g., co-culture with MS5-DLL1/4 stromal cells, OP9 or OP9-DLL1 stromal cells, or EpCAM-CD56⁺ stromal cells) described in WO2021/092581.

When the ATO method is performed, separating cells that can differentiate into CD4⁺ T cells may not be performed before the ATO method is performed, or the ATO method can also be performed on the separated cells that can differentiate into CD4⁺ T cells by a known method (e.g., flow cytometry or magnetic cell separation).

A nucleic acid for expressing a Notch ligand is preferably introduced into stromal cells. Stromal cell expressing a Notch ligand can be produced by introducing the nucleic acid into the stromal cells. Further, stromal cell expressing a Notch ligand can be produced by introducing the nucleic acid into pluripotent stem cells (particularly iPS cells (especially human iPS cells)) and then differentiating the pluripotent stem cells into stromal cells.

Examples of the stromal cells used in the production method of the present invention include stromal cells induced to differentiate from pluripotent stem cells (particularly iPS cells (especially human iPS cells)), and among them, it is desirable to use cells obtained by inducing pluripotent stem cells (particularly iPS cells (especially human iPS cells)) to differentiate into fibroblasts. The differentiation of pluripotent stem cells into stromal cells can be performed according to a known method. When pluripotent stem cells are human iPS cells, for example, fibroblasts induced to differentiate from iPS cells can be produced by the method described in PLoS ONE 8(10): e77673, 2013.

The Notch ligand is not particularly limited, and includes canonical Notch ligand and non-canonical Notch ligand described in WO2017/075389. Examples of the canonical Notch ligand include DLL4 (Delta-like ligand 4), DLL1 (Delta-like ligand 1), JAG1 (Jagged 1), JAG2 (Jagged 2), and the like. These can be used singly or in a combination of two or more types thereof. Examples of the non-canonical Notch ligand include Contactin-1, NOV/CCN3, Contactin-6, Periostin/OSF-2, DLK2/EGFL9, Pref-1/DLK1/FA1, DNER, Thrombospondin-2, MAGP-1/MFAP2, Thrombospondin-3, MAGP-2/MFAP5, Thrombospondin-4, and Netrin-1. Human DLL4 is preferably used as the Notch ligand.

The means for introducing the nucleic acid for expressing the Notch ligand into stromal cells or pluripotent stem cells is not particularly limited, and various known or commonly used means can be adopted. Typically, the nucleic acid for expressing the Notch ligand is introduced into stromal cells using an expression vector to allow the cells to express the Notch ligand. The expression vector may be linear or cyclic, and may be a non-viral vector such as a plasmid, a viral vector, or a transposon vector.

The means for introducing the expression vector into stromal cells can be made appropriate according to the embodiment. For example, the expression vector can be introduced into stromal cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into stromal cells by a virus infection method. Examples of viral vectors include retroviral vectors, lentiviral vectors, adenoviral vectors, and adeno-associated viral vectors. When using these viral vectors, a vector containing the nucleic acid for expressing the Notch ligand and a packaging vector (plasmid) of each virus may be transfected into host cells using a corresponding commercially available kit to produce a recombinant virus, and then stromal cells may be infected with the obtained recombinant virus.

In addition to the nucleic acid for expressing the Notch ligand, the expression vector may contain sequences, such as nuclear localization signal (NLS) and multi-cloning site (MCS), if necessary. The expression vector may further contain a nucleic acid (base sequence) encoding a "functional gene," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The three-dimensional cell aggregate can be formed, for example, by centrifuging cells that can differentiate into CD4⁺ T cells and stromal cells expressing a Notch ligand. A nucleic acid for expressing a Notch ligand is preferably introduced into stromal cells. The ratio of the stromal cells to the cells that can differentiate into CD4⁺ T cells can be suitably adjusted by a person skilled in the art according to the type of stromal cells and cells that can differentiate into CD4⁺ T cells used, and the like, and is, for example, 100:1 to 1:100, 90:1 to 1:90, 80:1 to 1:80, 70:1 to 1:70, 60:1 to 1:60, 50:1 to 1:50, 40:1 to 1: 40, 30:1 to 1:30, 20:1 to 1:20, 10:1 to 1:10, and the like. For example, when the stromal cells are mouse bone marrow cells (particularly, MS-5), the ratio is preferably 20:1 to 1:4, and when the stromal cells are pluripotent stem cell-derived stromal cells (particularly, fibroblasts induced to differentiate from iPS cells (particularly human iPS cells)), the ratio is preferably 4:1 to 1:4 and further preferably 1:1.

The medium for culturing the three-dimensional cell aggregate is not particularly limited, and examples thereof include a serum-containing medium, a serum-free medium, or a xeno-free medium, and preferably include a serum-free medium, particularly a serum-free medium containing insulin (and further biotin, transferrin, and albumin).

Examples of the basal medium for culturing the three-dimensional cell aggregate include AIM V, X-VIVO-15, NeuroBasal, EGM2, TeSR, BME, BGJb, CMRL 1066, Glasgow's MEM, Improved MEM Zinc Option, IMDM, 199 medium, Eagle's MEM, αMEM, DMEM, Ham, RPMI-1640, Fischer's medium, and the like. These media may be used singly or as a mixture of two or more types thereof.

The medium may contain serum or may be serum-free. The medium may further contain serum replacements (e.g., albumin, transferrin, Knockout Serum Replacement (KSR), fatty acid, insulin, collagen precursor, minor element, 2-mercaptoethanol, 3'-thiolglycerol, ITS-supplement, B27 (trademark) supplement, etc.). Serum replacements can be used singly or in a combination of two or more types thereof.

The medium may further contain one or more substances of lipids, amino acids (non-essential amino acids etc.), L-glutamine, vitamins, growth factors, cytokines, antibiotics, antioxidants, pyruvic acid, buffers, inorganic salts, and the like. As the medium, it is desirable to use a chemically defined medium that does not contain materials with unknown components, such as serum, so as to reduce the difference in medium among lots and prepare cells with stable quality. In addition, a medium component described in WO2017/075389 and the like can be appropriately blended.

The culture temperature of the three-dimensional cell aggregate is, for example, 20 to 40°C, preferably about 37°C, the CO₂ concentration is, for example, 2% to 10%, preferably 2 to 5%, and the oxygen concentration is, for example, 1 to 20%, preferably 5 to 20%. The cell density at the start of culture can be, for example, about 1.0×10⁴ to 1.0x10¹⁰ cells/mL.

The culture period of the three-dimensional cell aggregate can be suitably adjusted by a person skilled in the art according to, for example, the type of cells for use, such as of stromal cells and cells that can differentiate into CD4⁺ T cells, and is, for example, 4 weeks or more, preferably 5 weeks or more, and more preferably 6 weeks or more. The upper limit of the culture period is not particularly limited, and is preferably 16 weeks or less, more preferably 14 weeks or less, further preferably 12 weeks or less, and particularly preferably 9 weeks or less.

Since cells other than CD4⁺ T cells may be contained in the cell population containing CD4⁺ T cells obtained by culturing a three-dimensional cell aggregate, CD4⁺ T cells may be isolated and recovered by, for example, flow cytometry (typically, fluorescence activated cell sorting: FACS) using a fluorescently labeled anti-CD4 antibody and a cell sorter with respect to the cell population containing CD4⁺ T cells. Further, CD4SP T cells may be isolated and recovered. Further, effector T cells (Tconv) that are fractionated to be CD25 negative may be isolated and recovered.

The cell population containing CD4⁺ T cells obtained by culturing a three-dimensional cell aggregate may undergo an expansion-culture step according to a known method (e.g., culturing the cell population containing CD4⁺ T cells in a medium containing an anti-CD3 antibody and IL-2) before step (1).

When a cell population containing CD8⁺ T cells is used in step (1), step (2) is performed for inducing differentiation of the cell population containing CD8⁺ T cells.

In the production method of the present invention, as the CD4⁺ T cells, cells having an expression construct (also referred to as "CNS-FOXP3" in the present specification) introduced may be used, the expression construct comprising:
(a) CNS1, CNS2, and CNS3 of FOXP3 gene;
(b) a promoter; and
(c) a nucleic acid encoding FOXP3.

The cells into which the expression construct is introduced are not particularly limited, and may be at any stage of differentiation, and examples thereof include CD4⁺ T cells, pluripotent stem cells, and cells that can differentiate into CD4⁺ T cells. Examples of the method for introducing the expression construct into CD4⁺ T cells, pluripotent stem cells, or cells that can differentiate into CD4⁺ T cells include the methods described above.

The expression construct in the present invention is for expressing FOXP3, and comprises (a) CNS1 (conserved non-coding sequence 1), CNS2 (conserved non-coding sequence 2), and CNS3 (conserved non-coding sequence 3) of FOXP3 gene, (b) a promoter, and (c) a nucleic acid encoding FOXP3. Regulatory T cells can be produced with high efficiency by introducing such an expression construct into CD4⁺ T cells, pluripotent stem cells, or cells that can differentiate into CD4⁺ T cells. The expression construct is not particularly limited as long as it can express FOXP3. The expression construct preferably contains a terminator, a polyadenylation signal, FOXP3 3'UTR, etc., in addition to (a), (b), and (c) mentioned above, and is more preferably one whose components such as these function in cells having the expression construct introduced thereinto.

The base sequences of human FOXP3 gene are registered as RefSeq Accession Nos. NM_001114377 (SEQ ID NO: 1) and NM_014009 (SEQ ID NO: 2), and the amino acid sequences thereof are also registered as RefSeq Accession Nos. NP_001107849 (SEQ ID NO: 3) and NP_054728 (SEQ ID NO: 4). These RefSeq IDs are registered on the NCBI website. In the present invention, the above gene also includes degenerate products and variants of genes other than those having the base sequences registered in the database mentioned above, and desirable variants are those encoding proteins having a biological activity equivalent to that of the protein consisting of the above amino acid sequences. Examples of variants include FOXP3 variants having an amino acid sequence that is 80% or more, 85% or more, 90% or more, 91% or more, 92% or more, 93% or more, 94% or more, 95% or more, 96% or more, 97% or more, 98% or more, or 99% or more, identical to the natural amino acid sequence. In the present specification, FOXP3 refers to a protein, and Foxp3 refers to a gene; however, FOXP3 and Foxp3 refer to a gene and protein, respectively, when such an interpretation is appropriate.

The nucleic acid encoding FOXP3 is not particularly limited as long as it is a nucleic acid encoding FOXP3 protein, and preferably cDNA of FOXP3.

CNS1, CNS2, and CNS3 used in the present invention are all derived from FOXP3 gene. CNS1, CNS2, and CNS3 are FOXP3 enhancer elements. The promoter used in the present invention is not particularly limited, and examples include CAG promoter, ubiquitin gene promoter, FOXP3 gene promoter, EF1α promoter, SRα promoter, SV40 promoter, LTR promoter, cytomegalovirus (CMV) promoter, Rous sarcoma virus (RSV) promoter, Moloney murine leukemia virus (MoMuLV) LTR, herpes simplex virus thymidine kinase (HSV-TK) promoter, and the like; and preferably FOXP3 gene promoter. Preferred examples of the base sequences of CNS1, CNS2, CNS3, and promoter of human FOXP3 gene include the base sequences represented by SEQ ID NOs: 5 to 8, respectively. The promoter, CNS1, CNS2, and CNS3 include variants even if they do not have the base sequences described above, and desirable variants are those having a biological activity equivalent to that of the promoter, CNS1, CNS2, and CNS3 consisting of the above base sequences. Examples of variants include those having a base sequence that is 80% or more, 85% or more, 90% or more, 91% or more, 92% or more, 93% or more, 94% or more, 95% or more, 96% or more, 97% or more, 98% or more, or 99% or more, identical to the natural base sequence.

The arrangement (order) of the promoter, CNS1, CNS2, CNS3, and nucleic acid encoding FOXP3 in the expression construct is not particularly limited, and it is preferable that CNS1, CNS2, and CNS3 are located upstream of the promoter; and the order of CNS1, CNS2, CNS3, promoter, and nucleic acid encoding FOXP3 from the 5'-terminal side is more preferred.

The cells into which the expression construct is introduced are not particularly limited as long as they are CD4⁺ T cells, pluripotent stem cells, or cells that can differentiate into CD4⁺ T cells. Examples thereof include pluripotent stem cells, hematopoietic stem cells, hemogenic endothelial cells, hematopoietic progenitor cells, progenitor T cells, mesodermal progenitor cells, helper T cells, and the like, pluripotent stem cells are preferred, and iPS cells are more preferred. Of these, pluripotent stem cells (in particular, iPS cells) are preferably used as cells into which the expression construct is introduced in order to induce their differentiation into CD34⁺ cells (in particular, hemogenic endothelial cells) and perform the ATO method on these CD34⁺ cells (in particular, hemogenic endothelial cells), thereby differentiating them into regulatory T cells.

Regulatory T cells obtained by the production method and proliferation method of the present invention may be regulatory T cells into which a foreign gene is introduced.

The "foreign gene" is a gene to be introduced externally to express desired proteins in the regulatory T cells, and can be suitably selected according to the use of the regulatory T cells.

The foreign gene can be, for example, a gene for expressing a chimeric antigen receptor (CAR), and can further contain a gene for expressing a cytokine and/or a chemokine. As with general or known CARs, the CARs expressed by the regulatory T cells are basically configured such that peptides at sites of (i) an antigen recognition site that recognizes cell surface antigens of cancer cells (e.g., single-chain antibody), (ii) a transmembrane region, and (iii) a signal transduction region that induces the activation of T cells, are linked via a spacer, as needed. The foreign gene can also be, for example, a gene for expressing an exogenous T-cell receptor (TCR). The exogenous TCR means that it is exogenous to T cells into which a nucleic acid encoding the exogenous TCR is to be introduced. The amino acid sequence of the exogenous TCR may be identical to or different from that of the endogenous TCR of the T cells. One or two or more foreign genes may be introduced (e.g., CAR and exogenous TCR).

Examples of the means for introducing the foreign gene into cells include the methods mentioned above. The cells into which the foreign gene is introduced are not particularly limited, and may be at any stage of differentiation, and examples thereof include pluripotent stem cells (particularly, iPS cells), CD4⁺ T cells, regulatory T cells, and the like.

The cell population containing regulatory T cells produced by the production method or proliferation method of the present invention is useful for the treatment of animals (particularly, humans) with an abnormally enhanced immune response, and for example, is useful for the treatment and prevention of immune dysregulation, polyendocrinopathy, enteropathy, X-linked (IPEX) syndrome, graft-versus-host disease (GVHD), rejection in organ transplantation, autoimmune diseases, inflammatory diseases, allergic diseases (hay fever, asthma, atopic dermatitis, eczema, food allergy, food hypersensitivity, urticaria, allergic rhinitis, allergic conjunctivitis, and drug allergy), and the like, with no limitation thereto. The regulatory T cells produced by the production method of the present invention may be used for autologous transplantation or allogeneic transplantation. Further, the regulatory T cells may be used in combination with other drugs.

In such a cell therapy, from the viewpoint that rejection does not occur, the subject from which cells are isolated for use in the production of regulatory T cells preferably has the same HLA type as a subject to which regulatory T cells are to be administered, and is more preferably the same as the subject to which regulatory T cells are to be administered.

According to the present invention, a medicine comprising a cell population containing regulatory T cells (hereinafter also referred to as "the medicine of the present invention") can be produced. The medicine of the present invention is preferably produced as parenteral preparation by mixing an effective amount of regulatory T cells with a pharmaceutically acceptable carrier according to known means (e.g., the methods described in the Japanese Pharmacopoeia). The medicine of the present invention is preferably produced as a parenteral preparation, such as injections, suspensions, or drip infusions. Examples of parenteral administration methods include intravenous, intraarterial, intramuscular, intraperitoneal, or subcutaneous administration. Examples of pharmaceutically acceptable carriers include solvents, bases, diluents, excipients, soothing agents, buffers, preservatives, stabilizers, suspensions, isotonic agents, surfactants, solubilizing agents, and the like.

The dose of the medicine of the present invention can be suitably determined depending on various conditions, such as patient's body weight, age, sex, and symptoms, and in general, the medicine of the present invention is administered so that the number of cells per administration for a subject with a body weight of 60 kg is generally 1×10⁶ to 1x10¹⁰ cells, preferably 1×10⁷ to 1×10⁹ cells, and more preferably 5×10⁷ to 5×10⁸ cells. The medicine of the present invention may be administered once or several times. The medicine of the present invention can have a known form suitable for parenteral administration, such as injections or infusions. Further, the medicine of the present invention may contain physiological saline, phosphate buffered saline (PBS), medium, and the like in order to stably maintain the cells. Examples of the medium include RPMI, AIM-V, X-VIVO10, and other media, but are not limited thereto. In addition, pharmaceutically acceptable carriers (e.g., human serum albumin), preservatives, and the like may be added to the medicine for the purpose of stabilization. The medicine of the present invention is applied to mammals, including humans.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

### Examples

Examples will be given in the following to describe the present invention in more detail. However, the present invention is in no way limited to these Examples.

### Example 1: Expansion Culture of iPS-Tregs

The cells induced to differentiate from iPS cells into Tregs were expanded by culture in a medium containing IL-4 and TGF-β.

Specifically, the differentiation induction of iPS cells into Tregs is as follows: a plasmid sequence was designed and synthesized by introducing a DNA sequence obtained by changing mStrawberry protein sequence in the sequence (MK012431) registered in GenBank to dTomato sequence into a transfer plasmid for producing a third-generation lentiviral vector. The received plasmid was transfected into HEK293 lineage cells together with a packaging plasmid and an envelope plasmid for producing a lentiviral vector, and the supernatant containing the produced lentiviral vector was collected, and then concentrated by a high-speed centrifugation to obtain a lentiviral vector to be introduced into iPS cells. The Ff-I01s04 strain was seeded in a 24-well plate at 1×10⁴ cells/well, protamine was added at a final concentration of 10 µg/mL, and then a lentivirus suspension carrying CNS-FOXP3 gene was directly added to produce virally infected iPS cells.

The virally infected Ff-I01s04 strain was seeded at 1×10⁶ cells/well (Day 0) in a ultra-low attachment 6-well plate (Corning), in EB medium (StemPro34 (Gibco) supplemented with 50 ng/ml BMP4 (R&D Systems), 50 ng/ml bFGF (FUJIFILM Wako Pure Chemical Corporation), 50 ng/ml VEGF (R&D Systems), and 2 µM SB431542 (FUJIFILM Wako Pure Chemical Corporation), 10 µg/ml human insulin, 5.5 µg/ml human transferrin, 5 ng/ml sodium selenite (ITS, Gibco), 2 mM L-glutamine (Sigma-Aldrich), 45 mM α-monothioglycerol (Nacalai Tesque, Inc.), and 50 ug/ml ascorbic acid-2-phosphate (Sigma-Aldrich)), and then the cells were cultured under low-oxygen conditions (5% O₂) for 4 days (Day 4). Subsequently, the cells were further cultured for 4 days in a medium supplemented with 50 ng/ml bFGF, 50 ng/ml VEGF, and 50 ng/ml SCF (R&D Systems) (Day 8), thereby obtaining a cell population containing HECs. Furthermore, with stromal cell strain MS5 of mouse origin forced to express human DLL4 protein as a support, co-culture was performed with the CNS-FOXP3-transduced human iPS cell-derived HECs at a cell ratio of 4:1. The medium was RPMI-1640 (FUJIFILM Wako Pure Chemical Corporation) containing 2xB27 supplement (Invitrogen), 1xPSG (Sigma-Aldrich), 1xGlutamax (Invitrogen), 5 ng/mL IL-7 (PeproTech), 5 ng/mL FlT3L (PeproTech), and 50 µg/mL ascorbic acid (Sigma-Aldrich), at a final concentration, respectively, and the cells were co-cultured on 30 mm Millicell (hydrophilic PTFE, pore size: 0.4 µm, height: 5 mm, Merck Millipore) in a 6-well plate (TPP), and cultured for 9 weeks while changing the medium once every three or four days.

The cell population containing CNS-FOXP3-transduced human iPS cell-derived Tregs obtained above was seeded at 2.5×10⁵ cells/well in a 48-well cell culture plate coated with anti-CD3 antibodies (eBioscience) and cultured in a CO₂ incubator at 37°C with 5.0% CO₂ for 3 days. Then, the cells were transferred to a 24-well G-Rex cell culture plate for continued culture. The culture medium used was α-MEM (Invitrogen) containing the following components at final concentrations: FBS (15%, Corning), an L-glutamine-penicillin-streptomycin solution (1/100, Invitrogen, Sigma-Aldrich), insulin-transferrin-selenium supplement (1/100, Invitrogen), ascorbic acid-2-phosphate (50 µg/mL, Sigma-Aldrich), IL-2 (10 ng/mL, Peprotech), an anti-CD30 antibody (300 ng/mL, R&D systems), and a caspase inhibitor (10 µM, R&D systems). For the first 3 days, an anti-CD28 antibody (1.5 µg/mL, BioLegend) was additionally added to the composition above. The culture was performed for 14 days, with medium changes on day 3 from the start of culture and every 3 to 4 days thereafter. On day 10 of this culture period, CD4⁺ and CD4CD8 double-positive T cells were purified using CD4 MicroBeads (Miltenyi Biotec). The obtained cells were again expanded by culture under the same culture conditions as described above. On day 10, CD8-expressing cells were removed using CD8 MicroBeads (Miltenyi Biotec) to purify CD4⁺ T cells.

The cell population containing CD4⁺ T cells obtained above was seeded at 2.5×10⁵ cells/well in a 48-well cell culture plate coated with anti-CD3 antibodies (eBioscience) and cultured in a CO₂ incubator at 37°C with 5.0% CO₂ for 3 days. Then, the cells were transferred to a 24-well G-Rex cell culture plate for continued culture. The culture medium used was α-MEM (Invitrogen) containing the following components at final concentrations: FBS (15%, Corning), an L-glutamine-penicillin-streptomycin solution (1/100, Invitrogen, Sigma-Aldrich), an insulin-transferrin-selenium supplement (1/100, Invitrogen), ascorbic acid-2-phosphate (50 µg/mL, Sigma-Aldrich), IL-2 (10 ng/mL, Peprotech), an anti-CD30 antibody (300 ng/mL, R&D systems), a caspase inhibitor (10 µM, R&D systems), rapamycin (10 nM, Merck Millipore), and an anti-TNFR2 antibody (3 µg/mL, Hycult Biotech). For groups to which IL-3 (BioLegend), IL-4 (BioLegend), IL-33 (BioLegend), and/or TGF-β1 (BioLegend) were to be added, these substances were further added to the composition above in an amount of 60 ng/mL, 30 ng/mL, 30 ng/mL, and 5 ng/mL, respectively. For the first 3 days, an anti-CD28 antibody (1.5 µg/mL, BioLegend) was additionally added to the composition above. The culture was performed for 18 days, with medium changes on day 3 from the start of culture and every 3 to 4 days thereafter. The cell number was measured on days 3, 6, 10, 13, and 18, and a line graph was created. The results are shown in Fig. 1.

In the cell population with IL-4 and TGF-β1 added, cell proliferation was confirmed to have been promoted compared to the control (without IL-4 and TGF-β) (on day 18 of expansion culture, the cell count in the cell population with IL-4 and TGF-β added was 54.7-fold compared to that at the start of expansion culture, while the cell count of the control group was 38.4-fold). Cell populations with IL-3 or IL-33, or both, added in addition to IL-4 and TGF-β showed promoted cell proliferation to an equivalent or greater degree as compared to that in the group with only IL-4 and TGF-β added (on day 18, the group with additional IL-3 showed a 57-fold increase, the group with additional IL-33 showed an 87-fold increase, and the group with both IL-3 and IL-33 added showed a 77.8-fold increase).

### Example 2: Study of Protein Expression Levels in Tregs in Expansion Culture

Using the Tregs expanded by culture in Example 1, the expression levels of proteins expressed in Tregs were examined.

Specifically, each cell population on day 18 of expansion culture in Example 1 was stained using the following antibody sets and analyzed by flow cytometry:
Panel (1): Zombie NIR, BV510 CD3, BV421 CD4, PE/Cy7 CD8β, APC CD25, FITC FOXP3, PerCP/Cy5.5 CTLA4; and
Panel (2): Zombie NIR, BV510 CD3, BV421 CD4, PE/Cy7 CD8β, FITC FOXP3, PE Helios, PerCP/Cy5.5 CD39, APC CD73.

The results are shown in Fig. 2.

The cell populations with IL-4 and TGF-β added, as well as the cell populations with IL-3 added in addition to IL-4 and TGF-β, showed similar FOXP3 positive rates compared to the control (without IL-4 and TGF-β). The cell populations with both IL-3 and IL-33 added in addition to IL-4 and TGF-β, and the cell populations with only IL-33 added in addition to IL-4 and TGF-β, tended to show lower FOXP3 positive rates compared to the control. From the cell proliferation rates in Example 1, all groups were confirmed to have showed Treg (CD25⁺/FOXP3⁺ cells) proliferation to a greater degree as compared to the control, with the expression of proteins known as indicators of Treg suppressive function (Helios, CTLA4, CD39, and CD73) also maintained, indicating that the cells achieved favorable proliferation rates while maintaining Treg characteristics.

### Example 3: Evaluation of Suppressive Function of Tregs

Using Tregs expanded by culture in Example 1, analysis by flow cytometry after co-culture with human PBMC-derived allogeneic T cells was performed.

Specifically, T cells, derived from a donor different from that of Tregs, were treated with or without an anti-CD3 antibody and stained with CellTrace Violet (Thermo Fisher Scientific) to prepare target cells, and co-cultured with Tregs. The medium was α-MEM (Invitrogen) containing, FBS (15%, Corning), an L-glutamine-penicillin-streptomycin solution (1/100, Invitrogen, Sigma-Aldrich), an insulin-transferrin-selenium supplement (1/100, Invitrogen), and ascorbic acid-2-phosphate (50 µg/mL, Sigma-Aldrich), at a final concentration, respectively, and the cells were cultured for 4 days. Each cell population was stained with the following antibodies (Zombie NIR, PE/Cy7 CD3, and FITC HLA-A24). The results are shown in Fig. 3.

In the group co-cultured with iPS-Tregs, cell division of target cells was strongly suppressed in a Treg cell number dependent manner. Further, regarding the suppressive function, a stronger effect was observed in Tregs cultured with IL-3, IL-4, and TGF-β added as compared to the control (without IL-3, IL-4, and TGF-β). In the case of Treg:Target = 2:1, the suppression rate of the control group was 31.3%, whereas the suppression rate of 66.9% was observed in the cell population with IL-3, IL-4, and TGF-β added. These results indicate that Tregs sufficiently proliferated while maintaining their suppressive function.

### Example 4: Expansion Culture of iPS-Tregs

The cell populations containing CD4⁺ T cells obtained in Example 1 were seeded at 2×10⁵ cells/well in a 48-well cell culture plate coated with anti-CD3 antibodies (eBioscience) and cultured for 3 days in a CO₂ incubator at 37°C with 5.0% CO₂. The cells were then transferred to a 24-well G-Rex cell culture plate for continued culture. The culture medium used was α-MEM (Invitrogen) containing the following components at final concentrations: FBS (15%, Corning), an L-glutamine-penicillin-streptomycin solution (1/100, Invitrogen, Sigma-Aldrich), an insulin-transferrin-selenium supplement (1/100, Invitrogen), ascorbic acid-2-phosphate (50 µg/mL, Sigma-Aldrich), IL-2 (10 ng/mL, Peprotech), rapamycin (10 nM, Merck Millipore), anti-TNFR2 antibody (3 µg/mL, Hycult Biotech), IL-3 (60 ng/mL, BioLegend), IL-4 (30 ng/mL, BioLegend), IL-33 (30 ng/mL, BioLegend), and TGF-β1 (5 ng/mL, BioLegend). For control conditions, a medium without rapamycin, anti-TNFR2 antibody, IL-3, IL-4, IL-33, and TGF-β1 from the above medium composition was used. For the first 3 days, an anti-CD28 antibody (1.5 µg/mL, BioLegend) and an anti-CD30 antibody (300 ng/mL, R&D Systems) were additionally added to the above composition. The culture was performed for 14 days, with medium changes on day 3 from the start of culture and every 2 to 3 days thereafter. On day 14, a portion of the cells was seeded at 2×10⁵ cells/well in a 48-well cell culture plate coated with the anti-CD3 antibody and cultured again for 14 days in the same manner. This step was performed every 14 days for a total of 42 days of culture, and the cell number was measured to create a line graph. The results are shown in Fig. 4.

The cell populations obtained by expansion culture in a medium containing IL-3, IL-4, IL-33, and TGF-β1 demonstrated a stronger proliferation capacity than the cell population obtained by expansion culture under control conditions. An investigation using two lots of CNS-FOXP3-transduced iPS cells revealed that there was no difference in cell proliferation trends between batches. The group of expansion culture under control conditions did not proliferate sufficiently, and due to the low cell number, the accurate cell number could not be measured. Thus, expansion culture under control condition was terminated before day 42.

### Example 5: Study of Protein Expression Levels in Tregs in Expansion Culture

Using the cell populations expanded by culture in Example 4, the expression levels of proteins expressed in Tregs were examined.

Specifically, cell populations expanded by culture on day 8 and day 30 in Example 4 were stained with the following antibody set and analyzed by flow cytometry: Zombie NIR, BV510 CD3, BV421 CD4, PE/Cy7 CD8β, APC CD25, FITC FOXP3, PerCP/Cy5.5 CTLA4, PE Helios. The results are shown in Fig. 5.

In the group cultured with IL-3, IL-4, IL-33, and TGF-β1 added, the proportion of Tregs increased on day 8 compared to the group cultured under control conditions (27.5 to 28.9% in the control group, and 78.3 to 82.4% in the group cultured with IL-3, IL-4, IL-33, and TGF-β1). An investigation using cells differentiated from two lots of CNS-FOXP3-transduced iPS cells respectively revealed that there was also no difference in the expression levels of proteins in Tregs of the expansion culture cells.

### Example 6: Evaluation of Suppressive Function of Tregs

The cell populations expanded by culture in Example 4 were co-cultured with human PBMC-derived allogeneic T cells and then analyzed by flow cytometry.

Specifically, T cells from a different donor from the donor of Tregs, either untreated or treated with an anti-CD3 antibody, were stained with CellTrace Violet (Thermo Fisher Scientific) to prepare target cells and co-cultured with Tregs. The medium used was α-MEM (Invitrogen) containing the following components at final concentrations: FBS (15%, Corning), an L-glutamine-penicillin-streptomycin solution (1/100, Invitrogen, Sigma-Aldrich), an insulin-transferrin-selenium supplement (1/100, Invitrogen), and ascorbic acid-2-phosphate (50 µg/mL, Sigma-Aldrich). The cells were cultured in the α-MEM for 5 days. Each cell population was stained using the following antibody set (Zombie NIR, PE/Cy7 CD3, FITC HLA-A24). The results are shown in Fig. 6.

In the group co-cultured with iPS-Tregs, cell division of target cells was strongly suppressed in a Treg cell number-dependent manner. The suppressive function was similar between batches. These results indicated that Tregs sufficiently proliferated while maintaining their suppressive function.

### Example 7: Expansion Culture of iPS-Tregs

The cell populations containing CD4⁺ T cells obtained in Example 1 were seeded at 2×10⁵ cells/well in a 48-well cell culture plate coated with anti-CD3 antibodies (eBioscience) and cultured in a CO₂ incubator at 37°C with 5.0% CO₂ for 3 days. The cells were then transferred to a 24-well G-Rex cell culture plate for continued culture. The medium used was α-MEM (Invitrogen) containing the following components at final concentrations: FBS (15%, Corning), an L-glutamine-penicillin-streptomycin solution (1/100, Invitrogen, Sigma-Aldrich), an insulin-transferrin-selenium supplement (1/100, Invitrogen), ascorbic acid-2-phosphate (50 µg/mL, Sigma-Aldrich), IL-2 (10 ng/mL, Peprotech), an anti-TNFR2 antibody (3 µg/mL, Hycult Biotech), IL-3 (60 ng/mL, BioLegend), IL-4 (30 ng/mL, BioLegend), IL-33 (30 ng/mL, BioLegend), and TGF-β1 (5 ng/mL, BioLegend). For the first 3 days, an anti-CD28 antibody (1.5 µg/mL, BioLegend) and an anti-CD30 antibody (300 ng/mL, R&D systems) were further added to the composition above. The culture was performed for 14 days, with medium changes on day 3 from the start of culture and every 2 to 3 days thereafter. This step was repeated 4 times.

The obtained cell populations were further seeded at 2×10⁵ cells/well in a 48-well cell culture plate coated with anti-CD3 antibodies (eBioscience) and cultured in a CO₂ incubator at 37°C with 5.0% CO₂ for 3 days. The cells were then transferred to a 24-well G-Rex cell culture plate for continued culture. The medium used was α-MEM (Invitrogen) containing the following components at final concentrations: FBS (15%, Corning), an L-glutamine-penicillin-streptomycin solution (1/100, Invitrogen, Sigma-Aldrich), an insulin-transferrin-selenium supplement (1/100, Invitrogen), ascorbic acid-2-phosphate (50 µg/mL, Sigma-Aldrich), IL-2 (10 ng/mL, Peprotech), rapamycin (10 nM, Merck Millipore), an anti-TNFR2 antibody (3 µg/mL, Hycult Biotech), IL-3 (60 ng/mL, BioLegend), IL-4 (30 ng/mL, BioLegend), IL-33 (30 ng/mL, BioLegend), and TGF-β1 (5 ng/mL, BioLegend). Additionally, a CDK8/19 inhibitor (AS2863619, MedChemExpress) was added at 0.05 µg/mL, 0.015 µg/mL, 0.5 µg/mL, 1.5 µg/mL, or 5.0 µg/mL. For the first 3 days, an anti-CD28 antibody (1.5 µg/mL, BioLegend) and an anti-CD30 antibody (300 ng/mL, R&D systems) were further added to the composition above. The culture was performed for 17 days, with medium changes on day 3 from the start of culture and every 2 to 3 days thereafter. The cell number was measured and plotted on a line graph. The results are shown in Fig. 7.

The group with the CDK8/19 inhibitor added showed enhanced cell proliferation in a manner dependent on the concentration of the CDK8/19 inhibitor (on day 17 of expansion culture, cells with the 5.0 µg/mL CDK8/19 inhibitor added proliferated 58-fold compared to those before expansion culture, while the group without the CDK8/19 inhibitor proliferated 2.7-fold).

### Example 8: Study of Protein Expression Levels in Tregs in Expansion Culture

Using the cell populations expanded by culture in Example 7, the expression levels of proteins expressed in Tregs were examined. Specifically, cell populations expanded by culture on day 17 in Example 7 were stained with the following antibody set and analyzed by flow cytometry: Zombie NIR, BV510 CD3, BV421 CD4, PE/Cy7 CD8β, APC CD25, FITC FOXP3, PerCP/Cy5.5 CTLA4. The results are shown in Fig. 8.

The cell populations obtained through expansion culture in Example 7 showed an increase in the number of Tregs in a CDK8/19 inhibitor-dependent manner. In this case, the number of Tregs reached a plateau when the concentration of the CDK8/19 inhibitor was 0.15 µg/mL, and there was little increase in the number of Tregs even when the concentration was increased further. The cells treated with the CDK8/19 inhibitor also tended to show higher rates of CTLA4 positivity compared to the control.

The present application is based on Japanese Patent Application No. 2022-152606, filed on September 26, 2022, in Japan, the disclosure of which is incorporated herein by reference in its entirety.

Sequence Listing

## Claims

1. A method for producing a cell population in which regulatory T cells have proliferated, comprising:
(1) culturing a cell population containing CD4⁺ T cells derived from pluripotent stem cells in the presence of IL-4 and a TGF-βR agonist.

2. The method according to claim 1, wherein step (1) is performed in the presence of IL-4, a TGF-βR agonist, and IL-3.

3. The method according to claim 1, wherein step (1) is performed in the presence of IL-4, a TGF-βR agonist, and IL-33.

4. The method according to claim 1, wherein step (1) is performed in the presence of IL-4, a TGF-βR agonist, IL-3, and IL-33.

5. The method according to claim 1, wherein step (1) is further performed in the presence of at least one selected from the group consisting of IL-2, a TNFR2 agonist, and an mTOR inhibitor.

6. The method according to claim 5, wherein step (1) is further performed in the presence of IL-2, a TNFR2 agonist, and an mTOR inhibitor.

7. The method according to claim 1, wherein step (1) is performed in the presence of IL-4, a TGF-βR agonist, IL-3, IL-33, IL-2, a TNFR2 agonist, and an mTOR inhibitor.

8. The method according to claim 1, wherein step (1) is further performed in the presence of a CDK8 and/or CDK19 inhibitor.

9. The method according to claim 1, wherein the TGF-βR agonist is TGF-β.

10. The method according to any one of claims 5 to 7, wherein the mTOR inhibitor is rapamycin.

11. The method according to any one of claims 5 to 7, wherein the TNFR2 agonist is a TNFR2 agonist antibody.

12. The method according to claim 8, wherein the CDK8 and/or CDK19 inhibitor is at least one selected from the group consisting of 4-[1-(2-methyl-1H-benzimidazol-5-yl)-1H-imidazo[4,5-c]pyridin-2-yl]-1,2,5-oxadiazole-3-amine, 3-{1-[1-(4-methoxyphenyl)piperidin-4-yl]-4-methyl-1H-imidazo[4,5-c]pyridin-2-yl}pyrazine-2-amine, and siRNA of CDK8 and/or CDK19.

13. The method according to claim 1, wherein the regulatory T cells are CD25⁺/FOXP3⁺ cells.

14. The method according to claim 1, wherein the CD4⁺ T cells are at least one type of cells selected from the group consisting of CD25⁺/FOXP3⁺ cells and CD25⁻/FOXP3⁻ cells.

15. The method according to claim 1, wherein the CD4⁺ T cells have an expression construct introduced, the expression construct comprising
(a) conserved non-coding sequence (CNS) 1, CNS2, and CNS3 of FOXP3 gene,
(b) a promoter, and
(c) a nucleic acid encoding FOXP3.

16. The method according to claim 1, further comprising before step (1)
(2) inducing differentiation of pluripotent stem cells into a cell population containing CD4⁺ T cells.

17. The method according to claim 16, further comprising before step (2)
(3) introducing an expression construct into pluripotent stem cells,
the expression construct comprising
(a) CNS1, CNS2, and CNS3 of FOXP3 gene,
(b) a promoter, and
(c) a nucleic acid encoding FOXP3.

18. A method for proliferating regulatory T cells, comprising (1A) culturing a cell population containing regulatory T cells derived from pluripotent stem cells in the presence of IL-4 and a TGF-βR agonist.

19. A method for producing a cell population containing regulatory T cells, comprising (1B) culturing a cell population containing CD4⁺/CD25⁻/FOXP3⁻ T cells derived from pluripotent stem cells in the presence of IL-4 and a TGF-βR agonist.

20. The method according to claim 1, 18, or 19, wherein the pluripotent stem cells are iPS cells.

21. A cell population containing regulatory T cells, obtained according to the method of claim 1, 18, or 19.

22. A medicine comprising the cell population containing regulatory T cells of claim 21.

23. The medicine according to claim 22, for use in prevention and/or treatment of an abnormally enhanced immune response.

24. A method for preventing and/or treating an abnormally enhanced immune response, comprising administering the cell population containing regulatory T cells of claim 21 to a subject in need thereof.

25. The cell population containing regulatory T cells according to claim 21, for use in prevention and/or treatment of an abnormally enhanced immune response.

26. Use of the cell population containing regulatory T cells of claim 21 in the production of a medicine for use in prevention and/or treatment of an abnormally enhanced immune response.
